(19) Europäisches Patentamt
European Patent Office
Office européen des brevets

(11) Veröffentlichungsnummer: **0 120 321**
**B1**

(12) # EUROPÄISCHE PATENTSCHRIFT

(45) Veröffentlichungstag der Patentschrift: **05.10.88**

(21) Anmeldenummer: **84102012.6**

(22) Anmeldetag: **27.02.84**

(51) Int. Cl.⁴: **C 07 D 295/18**, C 07 C 103/30, C 07 D 265/30, C 07 D 317/60, C 07 D 213/56, C 07 D 307/54, C 07 D 333/24, C 07 D 295/22 // C07C59/64, C07C69/734, C07C101/46, C07C103/44, C07C97/10, C07C79/36, C07C49/83

(54) **Acrylsäureamide, ihre Herstellung und Verwendung.**

(30) Priorität: **28.02.83 DE 3306996**
**07.03.83 DE 3308045**

(43) Veröffentlichungstag der Anmeldung: **03.10.84 Patentblatt 84/40**

(45) Bekanntmachung des Hinweises auf die Patenterteilung: **05.10.88 Patentblatt 88/40**

(84) Benannte Vertragsstaaten: **AT BE CH DE FR GB IT LI NL SE**

(56) Entgegenhaltungen:
**CA - A - 66 011 443**
**CA - A - 69 026 954**
**CA - A - 69 087 038**
**CA - A - 76 140 385**
**DE - A - 1 222 926**
**DE - A - 1 518 753**
**DE - B - 1 189 997**
**FR - A - 1 259 830**
**US - A - 4 342 781**

(73) Patentinhaber: **Shell Agrar GmbH & Co. KG, Binger Strasse 173, D-6507 Ingelheim am Rhein (DE)**

(72) Erfinder: **Nickl, Josef, Dr. Dipl.-Chem., Silcherstrasse 8, D-7950 Biberach an der Riss (DE)**
Erfinder: **Pieper, Helmut, Dr. Dipl.-Chem., Kapellenweg 5, D-7950 Biberach an der Riss (DE)**
Erfinder: **Curtze, Jürgen, Dr. Dipl.-Chem., Rheingaublick 6, D-6222 Geisenheim-Johannisberg (DE)**
Erfinder: **Drandarevski, Christo, Dr., Boehringerstrasse 8, D-6507 Ingelheim am Rhein (DE)**
Erfinder: **Lust, Sigmund, Dr., Klappacher Strasse 2 f, D-6100 Darmstadt (DE)**

(74) Vertreter: **Hunter, Keith Roger Ian et al, 4 York Road, London SE1 7NA (GB)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue Acrylsäureamide, ihre Herstellung und ihre Verwendung als Fungizide.

Aus der DE-Al 1 518 753 sind β-Methyl- und β-Ethyl-3,4,5-trimethoxyzimtsäureamide bekannt, die neben Tranquilizer-Eigenschaften auch eine Wachstumshemmung bei bestimmten Protozoen zeigen. Eine fungistatische bzw. fungizide Wirkung auf phytopathogene Pilze ist von diesen Verbindungen nicht bekannt.

Aus der US-Patentschrift 4 342 781 sowie aus einigen Publikationen (vgl. Chem. Abstr. 66, 11443, 69, 26954, 69, 87038, 76, 140385) sind verschiedene substituierte β-Phenylzimtsäureamide bekannt, die z.T. als Arzneistoffe, UV-Stabilisatoren und Antimalariamittel bezeichnet worden sind. Es findet sich jedoch auch im Zusammenhang mit diesen Verbindungen kein Hinweis auf das erfindungsgemässe Anwendungsgebiet.

Es war daher überraschend, dass die erfindungsgemässen Verbindungen eine z.T. hervorragende Wirkung gegen phytopathogene Pilze zeigen, insbesondere gegen falschen Mehltau. Die bei Fungiziden bisher nicht bekannte Struktur der neuen Verbindungen lässt erwarten, dass auch gegen andere Fungizide resistent gewordene phytopathogene Pilze erfolgreich bekämpft werden können.

Die neuen Acrylsäureamide haben die Formel

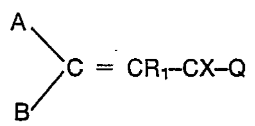 (I),

in der

A für die Gruppe

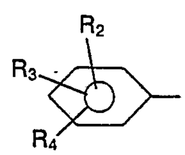 (II),

B für die Gruppe

$$Y-(CR_5=CR_6)_k- \quad (III),$$

Q für eine der Gruppen

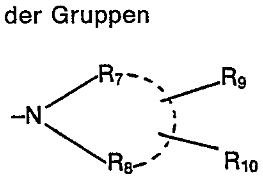 (IV)

und

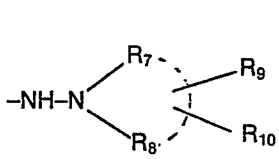 (V)

X für 0, S oder NH,

Y für $C_3$–$C_{10}$-Alkyl sowie zusätzlich für $C_1$–$C_2$-Alkyl, wenn k gleich 1 oder 2 ist, oder für substituiertes $C_1$–$C_{10}$-Alkyl oder für einen gegebenenfalls substituierten Rest aus der Gruppe $C_3$–$C_7$-Alkenyl, $C_3$–$C_7$-Cycloalkyl, $C_5$–$C_7$-Cycloalkenyl, Pyridyl, Furyl, Thienyl, α- und β-Naphthyl, wobei die Substituenten Halogen, Nitro, Amino, gegebenenfalls ein- oder mehrfach halogensubstituierte $C_1$–$C_4$-Alkyl- und Alkoxygruppen, $-NH(C_1–C_4\text{-Alkyl})$, $-N(C_1–C_4\text{-Alkyl})_2$, Phenoxy, Phenylthio oder $C_1$–$C_4$-Alkylthio sind; oder zusammen mit der Gruppe $CR_5$ für einen 5- bis 7-gliedrigen, vorzugsweise gesättigten, cycloaliphatischen Kohlenwasserstoffrest, an den ein Benzolring ankondensiert sein kann; oder für den Rest

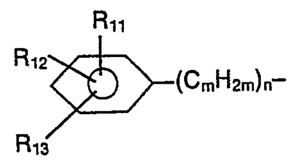 (VI)

steht, wobei

k die Zahlen 0, 1 oder 2,

m die Zahlen 1, 2, 3 oder 4,

n die Zahlen 0 oder 1,

$R_1$ Wasserstoff, $C_1$–$C_4$-Alkyl, Cyano und, wenn k gleich 0 ist, auch Chlor, Brom oder Jod,

$R_2$ und $R_{11}$ Wasserstoff, Halogen, Nitro, gegebenenfalls ein- oder mehrfach durch Fluor oder Chlor substituiertes $C_1$–$C_4$-Alkyl oder -Alkoxy, $C_3$–$C_4$-Alkenyloxy, $C_3$–$C_4$-Alkinyloxy, Amino, $-NH(C_1–C_4\text{-Alkyl})$, $-N(C_1–C_4\text{-Alkyl})_2$, Cyano, Phenyl, $C_3$–$C_6$-Cycloalkyl, Hydroxy($C_1$–$C_4$-Alkyl), $-NH-COR_6$, $-NH-COR_6$, $-CONR_7R_8$, durch Sauerstoff unterbrochenes $C_2$–$C_8$-Alkyl oder

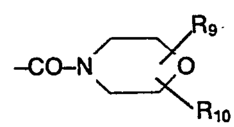 (VII),

$R_3$, $R_4$, $R_{12}$ und $R_{13}$ Wasserstoff, Halogen, $C_1$–$C_4$-Alkyl, $C_1$–$C_4$-Alkoxy, $(C_1–C_4\text{-Alkyl})-S(O)_p$ (p = 0, 1 oder 2), Hydroxy oder ($C_1$–$C_4$-Acyl)oxy, $R_3/R_4$ und $R_{12}/R_{13}$ jeweils gemeinsam auch Methylendioxy oder Ethylendioxy, gebunden an zwei benachbarte Atome des Phenylrings,

$R_5$ und $R_6$ Wasserstoff oder $C_1$–$C_4$-Alkyl, $R_5$ ausserdem, zusammen mit dem C-Atom, an das es gebunden ist, und Y einen 5- bis 7-gliedrigen, vorzugsweise gesättigten, cycloaliphatischen Rest, an den ein Benzolring ankondensiert sein kann.

$R_7$ und $R_8$ $C_1$–$C_4$-Alkyl, $C_3$–$C_7$-Cycloalkyl, Phenyl, Benzyl, Furfuryl, Tetrahydrofurfuryl oder $C_3$–$C_4$-Alkenyl, gemeinsam ausserdem auch eine $C_3$–$C_5$-Alkylenkette, die durch O, $NR_6$, $S(O)_q$ (mit q = 0, 1 oder 2) unterbrochen sein kann, $R_7$ ausserdem Wasserstoff,

$R_9$ und $R_{10}$, die nur vorliegen, wenn $R_7$ und $R_8$ gemeinsam eine Kette darstellen, Wasserstoff oder $C_1$–$C_4$-Alkyl bedeuten,

sowie gegebenenfalls die Säureadditionssalze der vorstehend definierten Verbindungen, mit der Einschränkung, dass A und B nicht beide für einen Rest aus der Gruppe Phenyl, Monohalogenphenyl, Mononitrophenyl und Monoaminophenyl stehen.

Im Rahmen der vorstehenden Definitionen können die Reste und Gruppen jeweils gleich oder verschieden sein.

Die Kohlenstoffketten der Alkyl-, Alkoxy-, Alkyl-$S(O)_p$-, Mono- oder Dialkylaminogruppen enthalten vorzugsweise 1–3, insbesondere 1–2 C-Atome. Weisen die Ketten mehr als 2 C-Atome auf, so können sie unverzweigt oder verzweigt sein. «Halogen» steht für Fluor, Chlor, Brom und Jod, vor allem für Chlor und Brom.

Der Rest A ist bevorzugt di- oder tri-substituiert, wobei im Falle von Disubstitution die zwei Substituenten, z.B. Methyl, Methoxy, Ethyl, Ethoxy, Fluor, Chlor, Brom, $-CF_3$, $CF_2Cl$, $CF_3O-$, $CH_3S-$, $CH_3SO-$, $CH_3SO_2-$, $-NH_2$, $-NHCH_3$, $-N(CH_3)_2)$, $-O-CH_2-O$, $-O-C_2H_4-O-$, sich bevorzugt in 3,4-Stellung befinden.

Q bedeutet vorzugsweise den Rest der Formel IV, worin $R_7$ und $R_8$ gemeinsam eine gegebenenfalls unterbrochene Alkylenkette darstellen, so dass IV z.B. Gruppen der folgenden Art darstellt:

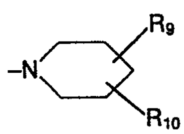 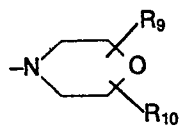 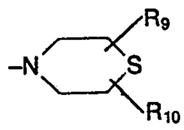 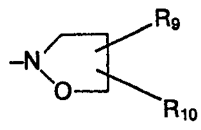

Sind A und B in der Formel I verschieden, so können die Verbindungen der Formel I als cis-/trans-Isomere vorliegen. Die Formel I umfasst in diesem Fall sowohl die einzelnen Isomeren als auch Gemische der cis- und der trans-Verbindung.

Man erhält die neuen Verbindungen nach an sich bekannten Verfahren.

a) Umsetzung einer Acrylsäure der Formel

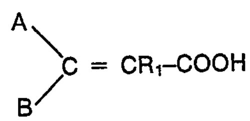 (VIII),

worin A, B und $R_1$ die obige Bedeutung haben, oder eines ihrer gegebenenfalls in situ hergestellten reaktionsfähigen Derivate mit einer Verbindung der Formel

$$H{-}Q \qquad (IX),$$

worin Q die obige Bedeutung hat, oder mit einem gegebenenfalls in situ hergestellten N-aktivierten Derivat von IX (wobei dann die Säure VIII selbst eingesetzt wird) umsetzt.

Das Verfahren ist somit eine Acylierung der Verbindung IX mit einer Carbonsäure der Formel VIII in Gegenwart eines die Säure aktivierenden oder eines wasserentziehenden Mittels oder mit reaktiven Derivaten der Säure oder die Umsetzung einer Carbonsäure der Formel VIII mit einer Verbindung der Formel IX in Gegenwart eines die Aminogruppe aktivierenden Mittels oder mit reaktiven Derivaten des Amins. Man erhält Endprodukte, in denen X Sauerstoff ist.

Als gegebenenfalls im Reaktionsgemisch hergestellte reaktive Derivate einer Carbonsäure der Formel VIII kommen beispielsweise ihre Alkyl-, Aryl-, Aralkylester oder -thioester wie der Methyl-, Ethyl-, Phenyl- oder Benzylester, ihre Imidazolide, ihre Säurehalogenide wie das Säurechlorid oder -bromid, ihre Anhydride, ihre gemischten Anhydride mit aliphatischen oder aromatischen Carbon-, Sulfen-, Sulfin-, Sulfonsäuren oder mit Kohlensäureestern, z.B. mit der Essigsäure, der Propionsäure, der p-Toluolsulfonsäure oder der O-Ethylkohlensäure, oder ihre N-Hydroxyimidester in Betracht. Als gegebenenfalls im Reaktionsgemisch hergestellte reaktive Derivate eines Amins der Formel IX eignen sich z.B. ihre «Phosphorazoderivate».

Die Art der Kohlenwasserstoffreste in den oben genannten Gruppen ist weitgehend unkritisch. Alkylgruppen enthalten im allgemeinen zwischen 1 und 12 C-Atome, können geradkettig oder verzweigt und sauerstoff- oder schwefelunterbrochen sein. Arylreste enthalten vorzugsweise 6 oder 10 C-Atome, Aralkylreste 7 bis 12 C-Atome, wobei der Alkylteil 1 bis 6 C-Atome enthalten und der aromatische Teil auch durch $C_1$–$C_3$-Alkyl substituiert sein kann (wobei dann der arylsubstituierte Alkylrest entsprechend weniger C-Atome umfassen würde).

Die aromatischen Ringe können auch gegebenenfalls gemischt substituiert sein, z.B. durch ein oder mehrere $C_1$–$C_2$-Alkylgruppen, $C_1$–$C_2$-Alkoxy und/oder ein oder mehrere Halogenatome.

Als säureaktivierende und/oder wasserentziehende Mittel kommen beispielsweise ein Chlorameisensäureester wie Chlorameisensäureäthylester, Phosphorpentoxid, N,N'-Dicyclohexylcarbodiimid, N,N'-Carbonyldiimidazol oder N,N'-Thionyldiimidazol in Betracht.

Die Umsetzung wird zweckmässigerweise in einem Lösungsmittel oder Lösungsmittelgemisch wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart einer anorganischen Base wie Natriumcarbonat oder einer tertiären organischen Base wie Triethylamin oder Pyridin, welche gleichzeitig als Lösungsmittel dienen kann, und gegebenenfalls in Gegenwart eines säureaktivierenden Mittels bei Temperaturen zwischen −25 °C und 150 °C, vorzugsweise jedoch bei Temperaturen zwischen −10 °C und der Siedetemperatur des Reaktionsgemisches, durchgeführt. Hierbei braucht ein gegebenenfalls im Reaktionsgemisch entstandenes reaktionsfähiges Derivat einer Verbindung der allgemeinen Formeln VIII oder IX nicht isoliert zu werden, ferner kann die Umsetzung auch in einem Überschuss der eingesetzten Verbindung der allgemeinen Formel IX als Lösungsmittel durchgeführt werden.

b) Umsetzung eines Ketons der Formel

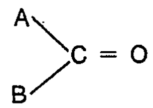 (X),

worin A und B die obige Bedeutung haben, mit einem Phosphonsäurederivat der Formel

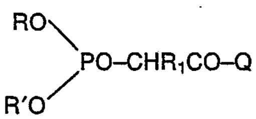 (XI),

in der $R_1$ und Q die obige Bedeutung haben und R und R′, die gleich oder verschieden sein können, für geradkettige oder verzweigte $C_1$–$C_{12}$-Alkylreste, für $C_7$–$C_{12}$-Aralkylreste oder $C_6$–$C_{10}$-Arylreste stehen. Die Alkylketten können durch Sauerstoff oder Schwefel unterbrochen sein, die Aryl- und Aralkylreste können ein- oder mehrfach durch Halogen, $C_1$–$C_2$-Alkyl oder -Alkoxy im Kern substituiert sein. Bevorzugt bedeuten R und R′ $C_1$–$C_3$-Alkylreste.

Die Umsetzung wird in Gegenwart eines basischen Stoffs wie Natriumhydrid, Kalium-tert-butylat, Natriummethylat oder Natriumamid, vorzugsweise in einem unter den Reaktionsbedingungen hinreichend inerten Lösungsmittel wie Tetrahydrofuran, Dioxan, 1,2-Dimethoxyethan, Dimethylformamid, Benzol, Toluol oder Gemischen solcher Lösungsmittel bei Temperaturen zwischen 0° und 150 °C, vorzugsweise zwischen 0 °C und der Siedetemperatur des Reaktionsgemischs durchgeführt. Als Reaktionsmedium kann auch ein Überschuss der Verbindung XI dienen.

c) Zur Herstellung solcher Verbindungen der Formel I, in denen X für Schwefel steht (Thioamide), werden die entsprechenden Amide in einem interten Lösungsmittel mit Phosphorpentasulfid umgesetzt.

Als Lösungsmittel eignen sich z.B. Toluol, Xylol, Benzol. Die Reaktionstemperatur liegt zwischen 0 °C und der Siedetemperatur des Reaktionsgemisches, wobei jedoch im allgemeinen eine Temperatur von 120 °C nicht überschritten wird.

d) Zur Herstellung solcher Verbindungen der Formel I, in denen X für NH steht (Amidine), werden entsprechende Iminoester der Formel

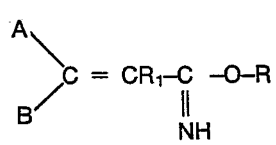 (XII),

worin A, B, R und $R_1$ die obige Bedeutung haben mit Aminen der Formel IX umgesetzt.

Die Umsetzung erfolgt in einem inerten Lösungsmittel wie Ether, Tetrahydrofuran bei Temperaturen zwischen etwa 0 und 100 °C.

e) Zur Herstellung solcher Verbindungen der Formel I, in denen k 0 (in Rest III) und $R_1$ Halogen, vor allem Chlor oder Brom bedeutet, wird bei einer entsprechenden Ausgangsverbindung an die Doppelverbindung der Acrylsäuregruppierung das Halogen addiert und anschliessend in der Wärme Halogenwasserstoff abgespalten.

Die Umsetzung erfolgt in inerten Lösungsmitteln wie Eisessig oder zweckmässig unter Zusatz von Eisessig bei Temperaturen zwischen etwa 30 und 100 °C.

f) Zur Herstellung von Verbindungen der Formel I, in denen $R_1$ CN bedeutet, können entsprechende Verbindungen mit $R_1$ gleich Halogen mit Kupfer(I)-cyanid in einem inerten Lösungsmittel umgesetzt werden.

Als inertes Lösungsmittel eignen sich beispielsweise Dimethylformamid, Dimethylsulfoxid. Die Reaktionstemperaturen betragen vorzugsweise zwischen 70 und 180 °C.

g) Zur Herstellung solcher Verbindungen der Formel I, in denen $R_1$ Cyan bedeutet, können auch Ketone der Formel X mit Cyanessigsäure-Derivaten der Formel

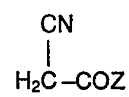 (XIII)

umgesetzt werden, worin Z für die Gruppe Q oder für die Gruppe OR stehen, wobei R wie oben definiert ist.

Die Umsetzung erfolgt in einem inerten Lösungsmittel, z.B. Benzol, Toluol, zweckmässig am Wasserabscheider und in Gegenwart eines Katalysators, z.B. Ammonacetat/Eisessig, in der Wärme, vorzugsweise bei der Siedetemperatur des Reaktionsgemischs. Bedeutet Z in der Verbindung XIII die Gruppe OR, so wird diese entsprechend Verfahren a) durch die Gruppe Q ersetzt.

h) Zur Herstellung solcher Verbindungen, in denen die Gruppen A und/oder B gegebenenfalls acylierte phenolische Hydroxygruppen enthalten, können entsprechende Verbindungen der Formel I, in denen die Gruppen A und/oder B niedere bis mittlere ($C_1$ bis $C_{12}$) Alkoxygruppen oder Benzyloxygruppen in der entsprechenden Position enthalten, einer Etherspaltung unterworfen und gegebenenfalls anschliessend acyliert werden.

Die Etherspaltung wird mit üblichen Mitteln, z.B. mit Bromwasserstoff/Eisessig, in der Wärme durchgeführt. Zur Acylierung dienen reaktionsfähige Derivate der einzuführenden Säuren, z.B. Säurechloride, Säureanhydride.

i) Zur Herstellung solcher Verbindungen der Formel I, in denen A und/oder B Aminogruppen enthalten, können entsprechende Nitroverbindungen nach üblichen Methoden reduziert werden.

Als Reduktionsmittel kann z.B. Natriumdithionit verwendet werden, als Reaktionsmedium ein inertes Lösungsmittel, z.B. Ethanol/Wasser. Die Reaktionstemperatur liegt zwischen Raumtemperatur und ca. 100 °C.

Erfindungsgemäss erhaltene cis-/trans-Isomerengemische können gewünschtenfalls anschlies-

send nach üblichen Methoden in die entsprechenden cis- und trans-Isomeren aufgetrennt werden.

Die Isomerentrennung erfolgt vorzugsweise durch fraktionierte Kristallisation, z.B. durch Kristallisation aus Methanol, Ethanol, Isopropanol, Methanol/Wasser oder Ethanol/Petrolether.

Verbindungen der Formel I mit basischen Gruppen können gewünschtenfalls in Säureadditionssalze übergeführt werden, vorzugsweise in Salze von Mineralsäuren wie Salzsäure, Bromwasserstoffsäure, Schwefelsäure, Phosphorsäure.

Die Ausgangsstoffe sind bekannt oder können nach üblichen Verfahren analog bekannten Verbindungen hergestellt werden, Verbindungen der Formel VIII, in denen B die Gruppe III darstellt und k 1 oder 2 bedeutet, beispielsweise nach dem folgenden Reaktionsschema:

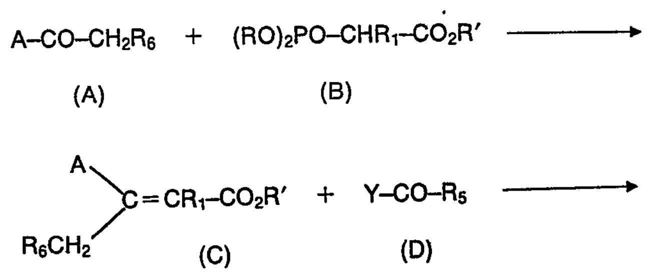

(A) (B) (C) (D)

VIII

Dabei haben A, R, R′, $R_1$, $R_5$, $R_6$ und Y die obige Bedeutung, R steht vorzugsweise für einen niederen Alkylrest, z.B. Methyl, Ethyl. Bevorzugt ist R′ ein $C_1$–$C_3$-Alkylrest. Der Olefinierungsschritt von B nach C kann z.B. nach der Horner-Variante der Wittig-Reaktion ausgeführt werden.

Ketone der Formel X erhält man beispielsweise durch Friedel-Craft-Acylierung eines entsprechenden Benzols mit 4-Nitrobenzoylchlorid, Reduktion der Nitrogruppe und anschliessende Halogenierung oder durch Umsetzung von Anilinhydrochlorid mit Methylal in Gegenwart von Acetanhydrid, Oxidation mit Chromtrioxid, Abspaltung der Acetylgruppen und anschliessende Halogenierung.

Ausgangsstoffe der Formel VIII, in denen A und B Phenylreste sind, können durch Umsetzung eines entsprechenden Benzophenons der Formel X mit einer Phosphonessigsäureverbindung der Formel

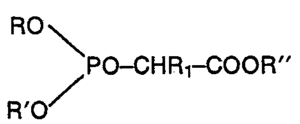 (XIV),

in der R, R′ und $R_1$ die obige Bedeutung haben und R″ für einen niederen Alkylrest steht, in Gegenwart einer Base und anschliessende Hydrolyse der Estergruppen erhalten werden.

Als Ausgangsstoffe benötigte Verbindungen der Formel

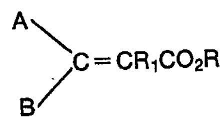 (XV),

(worin A, B, $R_1$ und R die obige Bedeutung haben), können auch nach der Reformatsky-Methode gemäss dem Schema

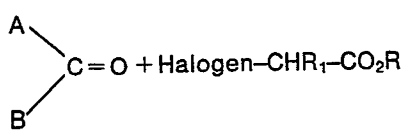 (XV)

gewonnen werden.

Ausgangsstoffe des Typs

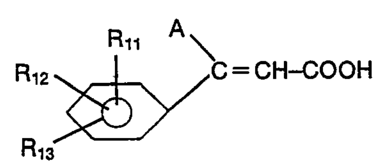 (XVI)

worin A, $R_{11}$, $R_{12}$ und $R_{13}$ die obige Bedeutung haben sind gemäss dem Schema

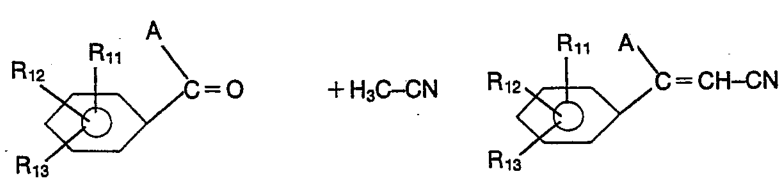

und anschliessende Verseifung erhältlich.

Die erfindungsgemässen Verbindungen zeigen eine starke Wirkung besonders gegen phytopathogene Pilze, vor allem gegen echten Mehltau, falschen Mehltau (etwa Plasmopara und Phytophthora), Schorf, Grauschimmel, Rostpilze. Wegen ihrer nur sehr geringen Phytotoxizität können die neuen Verbindungen in praktisch allen Nutz- und Zierpflanzenkulturen eingesetzt werden, beispielsweise in Getreide, etwa Mais, Weizen, Roggen, Hafer, in Reis, in Tomaten, Gurken, Bohnen, Kartoffeln, Rüben, im Wein- und Obstbau, in Rosen, Nelken und Chrysanthemen.

Die neuen Verbindungen zeigen Blattwirkung und systemische Wirkung. So wird mit zahlreichen erfindungsgemässen Verbindungen bei der Blattbehandlung gegen Plasmopara mit einer Wirkstoffkonzentration zwischen 20 und 100 ppm eine vollständige Abtötung der Pilze erreicht, (z.B. mit den Verbindungen nach Beispiel 1, 5, 11, 13, 21, 237, 37, 38, 43, 78, 90, 104, 105, 109, 121, 125, 127. Bei der Bekämpfung von Phytophthora genügen im allgemeinen Wirkstoffkonzentrationen von 100 ppm, zum Teil weniger, für eine ausreichende Wirkung (z.B. mit den Verbindungen nach Beispiel 6, 21, 24, 35, 37, 39, 40, 50, 65, 88, 101, 105, 106, 114, 125, 127, 130, 149, 157, 162.

Günstig ist es in manchen Fällen, die erfindungsgemässen Verbindungen mit bekannten fungiziden Wirkstoffen zu kombinieren. Dabei geht die Wirkung der Kombinationen z.T. deutlich über die rein additive Wirkung hinaus.

Kombinationspartner
Manganethylenbisdithiocarbamat (Maneb)
Mangan-Zinkethylenbisdithiocarbamat (Mancozeb)
Zinkethylenbisdithiocarbamat (Zineb)
N-Trichlormethylthio-tetrahydrophthalimid (Captan)
N-Trichlormethylthiophthalimid (Folpet)
N-(1,1,2,2-Tetrachlorethylthio)tetrahydrophthalimid (Captafol)
2,3-Dicyano-1,4-dithiaanthrachinon (Dithianon)
Zink-(N,N'-propylen-bisdithiocarbamat (Propineb)
Kupferoxychlorid
Natrium-4-dimethylaminobenzoldiazosulfonat (Fenaminosulf)
Triphenylzinnacetat (Fentinacetat)
Triphenylzinnhydroxid (Fentinhydroxyd)
Eisendimethyldithiocarbamat (Ferbam)
N-(2-Furoyl)-N-(2,6-xylyl)-DL-alanin (Furalaxyl)
3-(Dimethylamino)propylcarbamat (Propamocarb)
N-Ethyl-N-(3-dimethylamino)thiocarbamat (Prothiocarb)
Tetramethylthiuramdisulfid (Thiram)
N-Dichlorfluormethylthio-N,N'-dimethyl-N-p-tolylsulfamid (Tolylfluamid)
N-(2-Methoxyacetyl)-N-(2,6-xylyl)alanin (Metalaxyl)
Zinkdimethylthiocarbamat (Ziram)
N-Dichlorfluormethylthio-N,N'-dimethyl-N-phenylsulfamid (Dichlofluanid)
3-Trichlormethyl-5-ethoxy-1,2,4-thiadiazol (Etridazol)
Tri[aminzink-ethylenbis(dithiocarbamat)]tetrahydro-1,2,4,7-dithiadiazocin-3,8-dithion polymer (Metiram)
Aluminiumtris-(o-ethylphosphat) (Phosethyl)
2-Cyano-N-(ethylcarbamoyl)-2-methyloximino)-acetamid (Cymoxanil)
N-(3-Chlorphenyl)-N-(tetrahydrofuran-2-on-3-yl)-cyclopropancarbonamid (Cyprofuran)
Tetrachlor-isophthalodinitril (Chlorothalonil)
6-Methyl-2-oxo-1,3-dithio[4,5-b]-chinoxalin (Chinomethionat)
4-Cyclododecyl-2,6-dimethylmorpholin (Dodemorph)
1-Dodecylguanidinumacetat (Dodin)
Diisopropyl-5-nitroisophthalat (Nitrothal-isopropyl)
2,4-Dichlor-α-(pyrimidin-5-yl)-benzhydrylalkohol (Fenarimol)
1-(β-Allyloxy-2,4-dichlorphenethyl)imidazol (Imazalil)
3-(3,5-Dichlorphenyl)-N-isopropyl-2,4-dioxoimidazolidin-1-carboxamid (Iprodion)

Schwefel
2,3-Dihydro-6-methyl-5-phenylcarbamoyl-1,4-oxythiin-4,4-dioxid (Oxycarboxin)
N-(3,5-Dichlorphenyl)-1,2-dimethylcyclopropan-1,2-dicarboximid (Procymidon)
6-Ethoxycarbonyl-5-methylpyrazolo[1,5-]pyrimidin-2-yl-0,0-dimethylphosphorthioat (Pyrazophos)
2-(Thiazol-4-yl)-benzimidazol (Thiabendazol)
1-(4-Chlorphenoxy)-3,3-dimethyl-1-(1,2,4-triazol-1-yl)-2-butanon (Triadimefon)
1-(4-Chlorphenoxy)-3,3-dimethyl-1-(1,2,4-triazol-1-yl)-butanol (Triadimenol)
3-(3,5-Dichlorphenyl)-5-methyl-5-vinyloxyzolidin-2,4-dion (Vinclozolin)
Methylbenzimidazol-2-ylcarbamat (Carbendazin)
2,4,5-Trimethyl-N-phenyl-3-furancarboxamid (Methfuroxam)
β-[1,1'-Biphenyl]-4-yl-oxy)-λ-(1,1-dimethyl-ethyl)-1H-1,2,4-triazol-1-ethanol (Bitertanol)
2-(2-Furyl)benzimidazol (Fuberidazol)
5-Butyl-2-ethylamino-6-methylpyrimidin-4-ol (Ethirimol)
2-Methyl-3-furanilid (Fenfuram)
Bis-(β-guanidino-octyl)amin (Guazatin)
N-Cyclohexyl-N-methoxy-2,5-dimethylfuran-3-carbonsäureamid (Furmecyclox)

2-Chlor-4'-fluor-α-(pyrimidin-5-yl)benzhydrylalkohol (Nuarimol)
Methyl-1-(butylcarbamoyl)benzimidazolcarbamat (Benomyl)

0,0-Diethylphthalimidophosphonothioat (Dithalin)
7-Brom-5-chlorchinolin-8-yl-acrylat (Halacrimat)
1-/2-(2,4-Dichlorphenyl)-4-propyl-1,3-dioxolan-2-ylmethyl/1H-1,2,4-triazol (Propiconazol)
Dimethyl-4-4'-(o-phenylen)bis(3-thioallophanat) (Thiophanat-methyl)
1,4-Bis(2,2,2-trichlor-1-formamidoethyl)piperazin (Triforine)
2,6-Dimethyl-4-tridecylmorpholin (Tridemorph)
4-[3-[4-(1,1-Dimethyl-ethyl)phenyl]-2-methyl]-propyl-2,6(cis)-dimethylmorpholin (Fenpropemorph)
1,[2-(2,4-Dichlorphenyl)-4-ethyl-1,3-dioxolan-2-ylmethyl]1H-1,2,4-triazol (Etaconazol)
1-[1-(2,4-Chlorphenyl)-4,4-dimethyl-3-hydroxy-2-pentyl]1,2,4-triazol (Diclobutrazol)
2,4-Dichlor-6-(2-chloranilino)-1,3,5-triazin (Anilazin)

2-Jodo-N-phenylbenzamid (Benodanil)
2-sec.-butyl-4,6-dinitrophenyl-3-methylcrotonate (Binapacryl)
5-Butyl-2-(ethylamino)-6-methyl-4-pyrimidinyl dimethyl-sulfonat (Buprimat)
2,4-Dinitro-6-octylphenylcrotinat (Dinocap)
5,6-Dihydro-2-methyl-1,4-oxathiin-3-carbanilid (Carboxin)
N-Propyl-N-[(2,4,6-trichlorphenoxy)-2-ethyl]-imidazol-1-carbonamid (Prochloraz).

Für die Anwendung im Pflanzenschutz werden die neuen Verbindungen in üblicher Weise mit Hilfs- und/oder Trägerstoffen zu gebräuchlichen Formen von Schädlingsbekämpfungsmitteln verarbeitet, z.B. zu Lösungen, Emulsions- bzw. Lösungskonzentraten, Suspensionspulvern, Stäuben. Soweit Kombinationen mit anderen Wirkstoffen zur Anwendung gelangen sollen, kann dies in Form gemeinsamer Formulierungen oder z.B. in Form von Tankmischungen geschehen.

Die Konzentrate werden vor der Anwendung gegebenenfalls mit Wasser verdünnt, so dass Spritzbrühen mit einem Wirkstoffgehalt zwischen etwa 0,001 und 1 Gewichtsprozent erhalten werden. Bei der Anwendung als Low-volume- oder Ultra-Low-volume-Formulierung kann der Wirkstoffgehalt auch erheblich höher sein (bis ca. 20 bzw. bis ca. 90 Gewichtsprozent).

Beispiele für erfindungsgemässe Formulierungen:

1. Suspensionspulver
20 Gew.-Teile einer Verbindung der Formel I
20 Gew.-Teile Kaolin
5 Gew.-Teile Natriumsulfat
2 Gew.-Teile Schlämmkreide
9 Gew.-Teile Calciumligninsulfonat
1 Gew.-Teil Diisobutylnaphthalinnatriumsulfonat
43 Gew.-Teile Kieselkreide

Die Bestandteile werden vermahlen. Das Mittel wird für die Anwendung in so viel Wasser suspendiert, dass die Wirkstoffkonzentration etwa 0,001 bis 0,5 Gewichtsprozent beträgt.

2. Emulsionskonzentrat
15 Gew.-Teile einer Verbindung der Formel I
10 Gew.-Teile Dodecylbenzolsulfonsäuretriethylaminsalz
75 Gew.-Teile Dimethylformamid

Die nachstehenden Beispiele sollen die erfindungsgemässen Herstellungsverfahren näher erläutern.

Herstellung von Ausgangsprodukten:

4′-Chlor-4-nitro-benzophenon

Eine Mischung aus 100 g (0,54 Mol) 4-Nitrobenzoylchlorid, 100 g (0,75 Mol) Aluminiumchlorid und 100 ml = 111 g (0,99 Mol) Chlorbenzol wird unter Rühren und Ausschluss von Luftfeuchtigkeit auf 80 °C erhitzt, wobei sich eine Schmelze bildet. Nach Abklingen der Chlorwasserstoff-Bildung wird die Temperatur auf 100 °C gesteigert und bei dieser Temperatur $^1/_2$ Stunde lang weiter gerührt. Nach Abkühlen auf etwa 40–50 °C wird das zähflüssige Reaktionsgemisch auf Eis gegossen, wobei ein weisser Niederschlag ausfällt, der abgesaugt und mit Äthanol gewaschen wird.

Schmelzpunkt: 100–102 °C.

4-Amino-4′-chlor-benzophenon

Eine heisse Lösung von 13 g (0,05 Mol) 4′-Chlor-4-nitro-benzophenon in 80 ml Äthanol wird unter Rühren portionsweise zu einer heissen Lösung von 53,5 g (0,24 Mol) Zinn(II)chlorid-dihydrat in 50 ml konzentrierter Salzsäure unter heftiger Reaktion gegeben. Nach beendeter Zugabe erhitzt man während zwei Stunden auf dem Dampfbad und giesst anschliessend unter Rühren in wässrige Kalilauge. Es fällt ein Niederschlag aus, der abgesaugt und in siedendem Äthanol gelöst wird. Man filtriert vom Ungelösten und lässt erkalten. Die ausgeschiedenen hell gelben Kristalle werden abgesaugt.

Schmelzpunkt: 184–185 °C.

4-Amino-3,4′,5-trichlor-benzophenon

23,2 g (0,1 Mol) 4-Amino-4′-chlor-benzophenon werden in 200 ml Tetrahydrofuran gelöst. Zu dieser Lösung gibt man 400 ml Eisessig und gibt dann unter starkem Rühren und Eiskühlung schnell eine Lösung von 14,2 g (0,2 Mol) Chlor in 150 ml Eisessig zu. Man rührt weitere 5 Minuten und giesst auf Wasser. Es fällt ein weisser Niederschlag, der abgesaugt und aus Äthanol kristallisiert wird.

Schmelzpunkt: 165–167 °C.

4,4′-Diacetamino-diphenylmethan

Zu 260 g (2 Mol) Anilin-hydrochlorid, gelöst in 600 ml Wasser, tropft man unter Rühren 76 g (1 Mol) Methylal. Nach beendeter Zugabe erwärmt man eine Stunde auf 60 °C und anschliessend 3 Stunden auf 90 °C, wobei das sich bildende Methanol abdestilliert wird. Nach Abkühlen versetzt man die Lösung unter Eiskühlung mit einer konzentrierten Lösung von 40 g Natriumhydroxid. Es scheidet sich ein Öl ab, welches nach weiterem Rühren kristallisiert. Die Kristalle werden abgesaugt und zwischen 2 l Chloroform und 10 n Natronlauge verteilt. Die Chloroform-Lösung wird abgetrennt, über Natriumsulfat getrocknet und im Vakuum zur Trockne eingeengt. Der Rückstand wird in 1 l Benzol gelöst und solange mit Petroläther versetzt, bis sich ölige Verunreinigungen abscheiden. Nach Andekantieren wird die verbleibende Benzol-Petroläther-Lösung unter starkem Rühren in etwa 1 l Petroläther eingegossen, wobei 4,4′-Diamino-diphenyl-methan als Öl ausfällt. Diese fraktionierte Fällung wird noch 2 mal wiederholt. Das so gewonnene ölige 4,4′-Diamino-diphenyl-methan wird in möglichst wenig Eisessig gelöst und unter Rühren tropfenweise mit 205 g (2 Mol) Acetanhydrid versetzt und anschliessend eine Stunde auf 120 °C erwärmt. Nach dem Erkalten giesst man in etwa 3 l Wasser, saugt die ausgeschiedenen Kristalle ab und wäscht sie mit Wasser.

Schmelzpunkt: 228–230 °C.

4,4′-Diamino-benzophenon

Man löst 100 g Chromsäureanhydrid in 50 ml Wasser und füllt mit Eisessig auf 240 ml auf. 97 ml dieser Lösung gibt man unter Rühren und Kühlen langsam zu einer Lösung von 77 g 4,4′-Diacetamino-diphenylmethan, wobei man darauf achtet, dass die Temperatur nicht über 40 °C steigt. Danach wird unter weiterem Rühren während einer Stunde auf 90 °C erwärmt, anschliessend abgekühlt und in Eiswasser gegossen. Es fällt ein öliges Produkt aus, welches nach einigem Stehen kristallisiert. Die ausgefallenen Kristalle werden abgesaugt, mit Wasser gewaschen und anschliessend in 92 ml 66%iger Schwefelsäure 5 Minuten auf Rückflusstemperatur erhitzt. Nach dem Erkalten giesst man in Wasser und stellt die wässrige Lösung mit 10 n Natronlauge alkalisch, wobei rohes 4,4′-Diamino-benzophenon ausfällt.

Schmelzpunkt: 247–248 °C (Äthanol).

4,4′-Diamino-3,3′,5,5′-tetrachlor-benzophenon

25 g (0,12 Mol) 4,4′-Diamino-benzophenon werden in einer Mischung aus 25 ml konzentrierter Salzsäure, 200 ml Wasser und 500 ml Eisessig gelöst. Unter kräftigem Rühren und Kühlen mit Eiswasser versetzt man diese Lösung schnell mit einer Lösung von 33,5 g (0,42 Mol) Chlor in 400 ml Eisessig, rührt weitere 2 Minuten und giesst auf Eis. Der ausgefallene Niederschlag wird abgesaugt und aus Äthanol/Wasser kristallisiert.

Schmelzpunkt: 237–239 °C.

Vorbemerkung:

Das Isomere, dessen >C=C<u>H</u>–CO-Proton im NMR-Spektrum bei niederer Feldstärke erschien, wurde als Isomer A bezeichnet.

Beispiel 1

a) 4-Amino-β-(4-chlor-phenyl)-3,5-dichlor-zimtsäure-äthylester

Zu einer Suspension von 13,2 g (0,55 Mol) Natriumhydrid (26,4 g 50%ige Öl-Suspension) in 50 ml trockenem 1,2-Dimethoxy-äthan tropft man langsam unter Rühren und Eiskühlung eine Lösung von 108 g (0,55 Mol) Diäthylphosphonessigsäure-äthylester in 150 ml trockenem 1,2-Dimethoxy-äthan, wobei starkes Schäumen auftritt. Nach beendeter Zugabe wird weitere 30 Minuten gerührt. Anschliessend versetzt man mit einer Suspension von 150 g (0,5 Mol) 4-Amino-3,4′,5-trichlor-benzophenon in 800 ml trockenem 1,2-Dimethoxyäthan und erhitzt eine Stunde lang auf Rückflusstemperatur, wobei eine klare Lösung entsteht. Die Lösung wird im Vakuum zur Trockne eingeengt und der Rückstand zwischen Chloroform und Wasser verteilt. Die organische Phase wird getrocknet und im Vakuum zur Trockne eingeengt. Nach Versetzen mit 150 ml Äthanol tritt Kristallisation ein. Die Kristalle werden abgesaugt und mit Äthanol und Petroläther gewaschen. Schmelzpunkt des Isomerengemisches aus den Isomeren A und B: 88–110 °C.

NMR-Spektrum ($CDCl_3$, 60 MHz):

=C<u>H</u>–CO– 6,27 ppm Singulett } (1 Proton)
6,23 ppm Singulett }

Nach zweimaliger Umkristallisation aus Äthanol/Petroläther erhält man Kristalle des reinen Isomeren A vom Schmelzpunkt 124–125 °C.

NMR-Spektrum ($CDCl_3$, 60 MHz):

=C<u>H</u>–CO– 6,27 ppm Singulett (1 Proton)

b) 4-Amino-β-(4′-chlor-phenyl)-3-dichlor-zimtsäure

136 g (0,368 Mol) 4-Amino-β-(4′-chlor-phenyl)-3,4-dichlor-zimtsäure-äthylester (Isomerengemisch aus den Isomeren A und B) werden in einer Mischung aus 1000 ml Äthanol und 300 ml 5 n Natronlauge eine Stunde lang auf Rückflusstemperatur erhitzt. Nach dem Abkühlen verdünnt man mit Wasser und säuert mit 2n Salzsäure an. Es fällt ein zunächst öliges Produkt aus, das nach kurzer Zeit kristallisiert. Die Kristalle werden abgesaugt und aus Äthanol umkristallisiert. Man erhält farblose Kristalle des reinen Isomeren A vom Schmelzpunkt 241–243 °C (Zers.).

NMR-Spektrum (DMSO, 60 MHz):

=C<u>H</u>–CO– 6,37 ppm Singulett (1 Proton)

Durch weiteres Einengen der Mutterlauge wird eine weitere Kristall-Fraktion vom Schmelzpunkt 200–204 °C (Zers.) erhalten, die aus einem Gemisch der Isomeren A/B = 1/1,5 besteht.

NMR-Spektrum (DMSO, 60 MHz):

=C<u>H</u>–CO– 6,37 ppm Singulett } (1 Proton)
6,29 ppm Singulett }

c) 4-Amino-β-(4-chlor-phenyl)-3,5-dichlor-zimtsäure-morpholid

20 g (0,058 Mol) 4-Amino-β-(4-chlorphenyl)-3,5-dichlor-zimtsäure (Isomerengemisch der Isomeren A und B) im Verhältnis = 1 : 1,5) werden in 700 ml trockenem Chloroform gelöst. Unter Eiskühlung und Rühren gibt man bei 5 °C 5,93 g (0,058 Mol) Triäthylamin zu, kühlt auf −10 °C ab und versetzt mit 6,35 g (0,058 Mol) Chlorameisensäureäthylester. Nach beendeter Zugabe versetzt man unter weiterem Rühren und Kühlen mit 25,5 g (0,29 Mol) Morpholin. Man rührt weitere 4 Stunden bei Raumtemperatur, schüttelt anschliessend 3 mal mit Wasser aus, trocknet die organische Phase über Natriumsulfat und engt im Vakuum zur Trockne ein. Der Rückstand wird über eine Kieselgelsäure chromatographiert. (Kieselgel: Substanz = 10 : 1; Elutionsmittel: Chloroform/Essigester = 1 : 1). Die Substanz enthaltenden Eluate werden vereinigt und im Vakuum zur Trockne eingeengt. Man erhält farblose Kristalle vom Schmelzpunkt 140–144 °C, welche das cis-/trans-Isomeren-Gemisch im Verhältnis 3 : 2 enthalten.

NMR-Spektrum ($CDCl_3$, 80 MHz):

=C<u>H</u>–CO– 6,2 ppm Singulett } (1 Proton)
6,14 ppm Singulett }

Durch Umkristallisieren des erhaltenen cis-/trans-Isomeren-Gemisches aus Äthanol erhält man das reine trans-Isomer vom Schmelzpunkt 168–171 °C.

NMR-Spektrum ($CDCl_3$, 80 MHz):

=C<u>H</u>–CO– 6,2 ppm Singulett (1 Proton).

Die Aufklärung der sterischen Verhältnisse der so hergestellten Verbindung der Formel

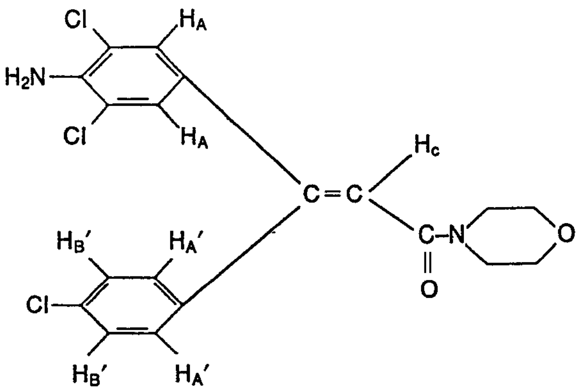

erfolgte durch $^1$H-NMR-Spektroskopie bei 90 MHz. Dieses zeigt die Signale

a) $H_A$ des 4-Amino-3,5-dichlor-phenyl-Ringes bei 7,09 ppm (Singulett)

b) $H_A'$ und $H_B'$ des 4-Chlorphenylringes bei 7,23 ppm (Dublett) und bei 7,33 ppm (Dublett)

c) $H_C$ des Olefins bei 6,22 ppm (Singulett)

(Das olefinische H der isomeren Verbindung (Isomer B) wird bei 6,17 ppm beobachtet, wie am Spektrum des Isomeren-Gemisches festgestellt wurde).

Zur Aufklärung der sterischen Verhältnisse der olefinischen Doppelbindung des obigen Moleküls wurden NOE-Messungen durchgeführt. (The Nuclear Overhauser Effect, J.H. Noggle, R.E. Schirmer, Academic Press New York und London 1971). Die NOE-Messungen erfolgten mit Hilfe eines 90 MHz-FT-NMR-Gerätes (Bruker Modell HX-90/15") an einer 2%igen Lösung der Substanz in besonders getrocknetem und entgastem $CDCl_3$.

Die NOE-Messung, homonuclear im $^1$H-NMR-Spektrum bei 90 MHz, erbrachte folgendes Ergebnis:

| bestrahltes Signal von H-Atom | beobachtetes Signal von H-Atom | Intensitätsänderung % |
|---|---|---|
| Hc (6,22 ppm) | $H_A$ (7,09 ppm) | +14 bis 15 |
| Hc (6,22 ppm) | $H_A'$ (7,23 ppm) | + 3 bis 5 |
| Hc (6,22 ppm) | $H_B'$ (7,33 ppm) | + 1 bis 3 |

Aus diesen Intensitätsmessungen (Tabelle) ergibt sich, dass der räumliche Abstand zwischen Hc und $H_A$ erheblich geringer ist als zwischen Hc und $H_A'$. Die Verbindung der obigen Formel liegt also in der dargestellten trans-Form vor.

Beispiel 2

4-Amino-β-(4'-chlor-phenyl)-3,5-dichlor-zimtsäure-morpholid

Zu 1 g Natriumhydrid (0,02 Mol, 50%ig in Öl), suspendiert in 50 ml trockenem Tetrahydrofuran, tropft man langsam unter Rühren eine Lösung von 5,3 g (0,02 Mol) Diäthylphosphonessigsäure-morpholid in 100 ml trockenem Tetrahydrofuran. Es tritt leichte Erwärmung und starkes Schäumen auf. Nach beendeter Zugabe rührt man weitere 10 Minuten. Zu der entstandenen klaren Lösung tropft man eine Lösung von 3 g (0,01 Mol) 4-Amino-3,4',5-trichlor-benzophenon in 20 ml trockenem Tetrahydrofuran und erhitzt anschliessend 16 Stunden lang auf Rückflusstemperatur. Nach Abkühlen giesst man ins Wasser und extrahiert erschöpfend mit Methylenchlorid. Die Methylenchlorid-Phase wird über Natriumsulfat getrocknet und im Vakuum eingeengt. Der Rückstand wird aus Isopropanol/Petroläther kristallisiert. Die erhaltenen farblosen Kristalle vom Schmelzpunkt 150–155 °C enthalten die cis-/trans-Isomeren im Verhältnis 1 : 3.

NMR-Spektrum ($CDCl_3$, 80 MHz):

=C<u>H</u>-CO- 6,2 ppm Singulett, 6,14 ppm Singulett (1 Proton)

Durch Umkristallisation des erhaltenen cis-/trans-Isomeren-Gemisches aus Äthanol erhält man reines trans-Isomer vom Schmelzpunkt 168–171 °C.

NMR-Spektrum ($CDCl_3$, 80 MHz):

=C<u>H</u>-CO- 6,2 ppm Singulett (1 Proton)

Beispiel 3

4-Amino-β-(4'-chlor-phenyl)-3,5-dichlor-zimtsäure-morpholid

Hergestellt aus 4-Amino-3,4',5-trichlor-benzophenon, Diäthylphosphonessigsäure-morpholid und Kalium-tert.-butylat in Dimethylformamid analog Beispiel 4.

Reinigung durch Säulenchromatographie und Kristallisation aus Isopropanol/Petroläther.

Schmelzpunkt des cis-/trans-Isomeren-Gemisches im Verhältnis 1 : 3: 150–155 °C,

NMR-Spektrum ($CDCl_3$, 80 MHz):

=C<u>H</u>-CO- 6,2 ppm Singulett, 6,14 ppm Singulett (1 Proton)

Beispiel 4

trans-4-Amino-β-(4'-chlor-phenyl)-3,5-dichlor-zimtsäure-morpholid

Hergestellt aus dem 4-Amino-β-(4'-chlor-phenyl)-3,5-dichlor-zimtsäure-Isomeren A mit Chlorameisensäure-äthylester und Morpholin analog Beispiel 3.

Schmelzpunkt: 168–171 °C,

NMR-Spektrum ($CDCl_3$, 80 MHz):

=C<u>H</u>-CO- 6,2 ppm Singulett (1 Proton)

Beispiel 5

4-Amino-β-(4'-amino-3',5'-dichlorphenyl)-3,5-dichlor-zimtsäure-morpholid

16,2 g (0,061 Mol) Diäthylphosphonessigsäure-morpholid werden in 100 ml trockenem Tetrahydrofuran gelöst. Unter Rühren gibt man portionsweise 1,5 g (0,061 Mol) Natriumhydrid (2,7 g 55%ige Öl-Suspension) zu. Nach beendeter Zugabe wird weitere 30 Minuten gerührt. Anschliessend versetzt man mit einer Lösung von 18 g (0,051 Mol) 4,4'-Diamino-3,3',5,5'-tetrachlorbenzophenon und erhitzt 18 Stunden lang auf Rückflusstemperatur. Nach dem Abkühlen giesst man die Lösung auf Eis/Wasser und extrahiert erschöpfend mit insgesamt 2 Ltr. Methylenchlorid. Die Methylenchlorid-Phase wird über Natriumsulfat und im Vakuum eingeengt. Der Rückstand wird in 200 ml Methylenchlorid suspendiert und kurz aufgekocht. Nach dem Erkalten wird die kristalline Substanz abgesaugt.

Schmelzpunkt: 256–258 °C.

Beispiel 6

4-Amino-β-(4-brom-phenyl)-3,5-dichlor-zimtsäuremorpholid

Hergestellt aus 4-Amino-β-(4′-brom-phenyl)-dibrom-zimtsäure (Isomerenverhältnis A : B = 1,5 : 1), Chlorameisensäureäthylester, Triäthylamin und Morpholin analog Beispiel 3.

Schmelzpunkt des 2,5 : 1-Gemisches aus den Isomeren A und B: 165–180 °C.

NMR-Spektrum ($CDCl_3$, 80 MHz):

=C$\underline{H}$–CO– 6,25 ppm Singulett, 6,20 ppm Singulett (1 Proton)

Beispiel 7

4-Amino-β-(4′-chlor-phenyl)-3,5-dibrom-zimtsäuremorpholid

Hergestellt aus 4-Amino-β-(4′-chlor-phenyl)-3,5-dibrom-zimtsäure (Isomerenverhältnis A : B = 5 : 1), Chlorameisensäureäthylester, Triäthylamin und Morpholin analog Beispiel 1c).

Schmelzpunkt des 6 : 1-Gemisches aus den Isomeren A und B: 178–189 °C.

NMR-Spektrum ($CDCl_3$, 80 MHz):

=C$\underline{H}$–CO– 6,22 ppm Singulett, 6,18 ppm Singulett (1 Proton)

Beispiel 8

4-Amino-3,5-dibrom-β-(4′-fluor-phenyl)-zimtsäuremorpholid

Hergestellt aus 4-Amino-3,5-dibrom-β-(4′-fluor-phenyl)-zimtsäure (Isomerenverhältnis A : B = 2,5 : 1), Chlorameisensäureäthylester, Triäthylamin und Morpholin analog Beispiel 1c).

Schmelzpunkt des 4 : 1-Gemisches aus den Isomeren A und B: 186–197 °C.

NMR-Spektrum ($CDCl_3$, 80 MHz):

=C$\underline{H}$–CO– 6,21 ppm Singulett, 6,16 ppm Singulett (1 Proton)

Beispiel 9

4-Amino-3,5-dibrom-β-phenyl-zimtsäuremorpholid

Hergestellt aus 4-Amino-3,5-dibrom-b-phenylzimtsäure (Isomerenverhältnis A : B = 1 : 1), Chlorameisensäureäthylester, Triäthylamin und Morpholin analog Beispiel 1c).

Schmelzpunkt des 1: 1-Gemisches aus den Isomeren A und B: 156–174 °C.

NMR-Spektrum ($CDCl_3$, 400 MHz):

=C$\underline{H}$–CO– 6,2 ppm Singulett, 6,18 ppm Singulett (1 Proton)

Beispiel 10

4-Amino-β-(4′-brom-phenyl)-3,5-dichlor-zimtsäuremorpholid

Hergestellt aus 4-Amino-β-(4′-brom-phenyl)-3,5-dichlor-zimtsäure (Isomerenverhältnis A : B = 1 : 2,5), Chlorameisensäureäthylester, Triäthylamin und Morpholin analog Beispiel 1c).

Schmelzpunkt des reinen Isomeren A: 188–194 °C.

NMR-Spektrum ($CDCl_3$, 60 MHz):

=C$\underline{H}$–CO– 6,23 ppm Singulett (1 Proton)

Beispiel 11

4-Amino-3,5-dichlor-β-(4′-fluor-phenyl)-zimtsäuremorpholid

Hergestellt aus 4-Amino-3,5-dichlor-β-(4′-fluorphenyl)-zimtsäure (Isomerenverhältnis A : B = 3 : 1), Chlorameisensäureäthylester, Triäthylamin und Morpholin analog Beispiel 1c).

Schmelzpunkt des 3 : 1-Gemisches aus den Isomeren A und B: 160–176 °C.

NMR-Spektrum ($CDCl_3$, 60 MHz):

=C$\underline{H}$–CO– 6,23 ppm Singulett, 6,18 ppm Singulett (1 Proton)

Beispiel 12

4-Amino-3,5-dichlor-β-phenyl-zimtsäuremorpholid

Hergestellt aus 4-Amino-3,5-dichlor-β-phenylzimtsäure (Isomerenverhältnis A : B = 1,5 : 1), Chlorameisensäureäthylester, Triäthylamin und Morpholin analog Beispiel 1c).

Schmelzpunkt des 1 : 1-Gemisches aus den Isomeren A und B: 143–160 °C.

NMR-Spektrum ($CDCl_3$, 400 MHz):

=C$\underline{H}$–CO– 6,2 ppm Singulett, 6,18 ppm Singulett (1 Proton)

Beispiel 13

a) 3-Brom-4-dimethylaminobenzophenon

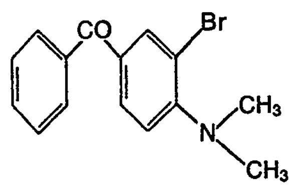

22,5 g (0,1 mol) 4-Dimethylaminobenzophenon werden in 60 ml Essigsäure gelöst. Unter Rühren wird innerhalb einer Stunde eine Lösung von 5,3 ml (0,105 mol) Brom in 20 ml Essigsäure zugetropft. Es wird 30 Minuten nachgerührt, mit Wasser versetzt und zur Entfärbung etwas Natriumhydrogensulfit zugegeben. Die dabei erhaltene Schmiere wird mit Toluol-Wasser geschüttelt, die Toluolphase noch zweimal mit Wasser gewaschen, getrocknet und das Toluol unter Vakuum abdestilliert.

Ausbeute: 24,4 g (80% d.Th.); zähes Öl.

b) 3-Brom-4-dimethylamino-β-phenylzimtsäure

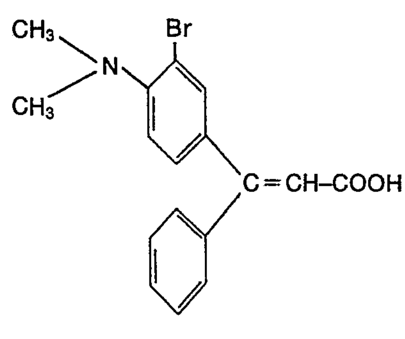

2,1 g (0,07 mol) Natriumhydrid (mit 20% Paraffinöl) werden mit 30 ml abs. 1,2-Dimethoxyethan gerührt. Unter Eiswasserkühlung wird eine Lösung von 15,7 g (0,07 mol) Triethylphosphonacetat in 30 ml 1,2-Dimethoxyethan zugetropft und nach klarer Lösung 18,25 g (0,06 mol) 3-Brom-4-dimethylaminobenzophenon, gelöst in 30 ml 1,2-Dimethoxyethan, zugegeben. Es wird 4 Stunden unter Rückfluss gekocht und nach dem Stehen über Nacht das Lösungsmittel unter Vakuum abdestilliert. Der Rückstand wird mit Toluol-Wasser geschüttelt, die Toluolphase nochmals mit Wasser gewaschen, getrocknet und das Toluol unter Vakuum abdestilliert. Der Rückstand wird durch zweistündiges Kochen unter Rückfluss mit methanolischer Kalilauge (aus 14 g (0,25 mol) Kaliumhydroxid und 250 ml Methanol + 25 ml Wasser) verseift. Die Lösung wird unter Vakuum eingedampft, der Rückstand mit Toluol-Wasser geschüttelt.

Die wässrige Lösung wird durch Zugabe von Salzsäure auf pH 6,5 eingestellt, wobei die Substanz in fester Form ausfällt. Diese ist in überschüssiger Salzsäure löslich. Nach dem Waschen mit Wasser wird getrocknet.

Ausbeute: 15,2 g (75% d.Th.);
Fp. 147 °C.

c) 3-Brom-4-dimethylamino-β-phenylzimtsäure-morpholid

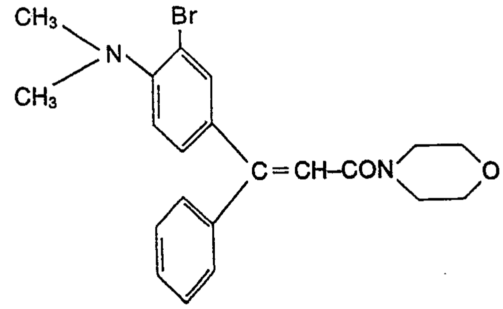

5,19 g (15 mmol) 3-Brom-4-dimethylamino-β-phenylzimtsäure werden in 30 ml abs. Tetrahydrofuran gelöst und portionsweise 3,24 g (20 mmol) 1,1'-Carbonyl-diimidazol zugegeben. Nach Beendigung der $CO_2$-Entwicklung und klarer Lösung werden 1,74 g (20 mmol) Morpholin zugesetzt, 10 Minuten bei Raumtemperatur stehen gelassen und anschliessend 30 Minuten unter Rückfluss gekocht. Die Lösung wird unter Vakuum eingedampft und der Rückstand mit Toluol-Wasser geschüttelt. Die Toluolphase wird noch zweimal mit Wasser gewaschen, getrocknet und unter Vakuum eingedampft. Das dabei erhaltene Öl kristallisiert beim Verreiben mit wenig Methanol.

Ausbeute: 6,25 g (91,5% d.Th.);
Fp. 169 °C.
Rf: 0,55 (Toluol-Aceton 70 : 30).

Entsprechend den obigen Beispielen können erfindungsgemäss auch die Verbindungen der nachstehenden Tabelle erhalten werden:

Verbindungen der Formel 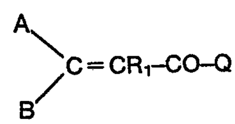

| Beispiel | A | B | Q | $R_1$ | Fp.(°C) | Rf |
|---|---|---|---|---|---|---|
| 14 | 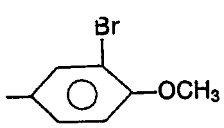 | $C_6H_5$ | 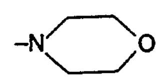 | H | Öl | 0,51* |
| 15 | 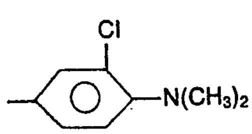 | $C_6H_5$ | 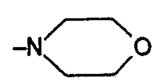 | H | | |
| 16 | 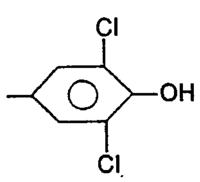 | $C_6H_5$ | 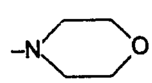 | H | 174 | |

| Beispiel | A | B | Q | $R_1$ | Fp.(°C) | Rf |
|---|---|---|---|---|---|---|
| 17 | 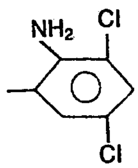 | $C_6H_5$ | 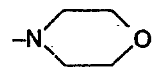 | H | 136 | |
| 18 | 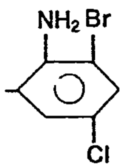 | $C_6H_5$ | 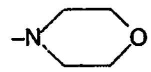 | H | 75–80 | |
| 19 | 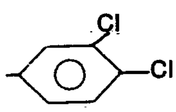 | 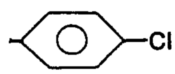 | 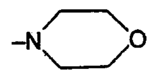 | H | Öl | |
| 20 | 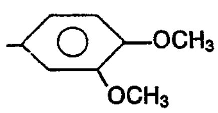 | 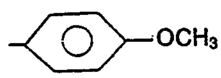 | 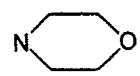 | H | | |
| 21 | 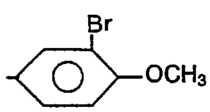 | 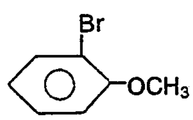 | 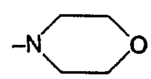 | H | 93 | |
| 22 | 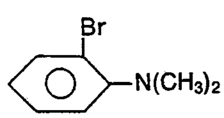 | 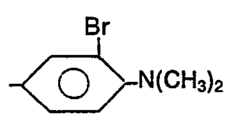 | 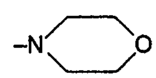 | H | Öl | 0,55* |
| 23 | 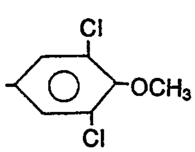 | $C_6H_5$ | 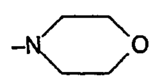 | H | Öl | 0,57* |
| 24 | 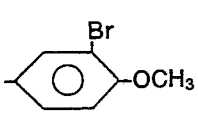 | $C_6H_5$ | 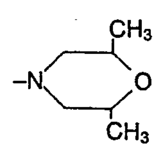 | H | Öl | 0,49 u. 0,59* |

| Beispiel | A | B | Q | $R_1$ | Fp.(°C) | Rf |
|---|---|---|---|---|---|---|
| 25 | 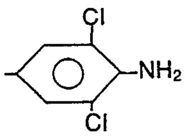 | 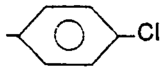 | 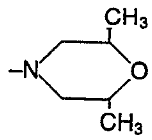 | H | Öl | 0,58* |
| 26 | 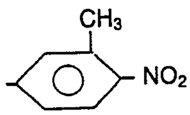 | $C_6H_5$ | 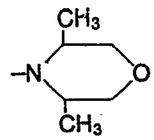 | H | | |
| 27 | 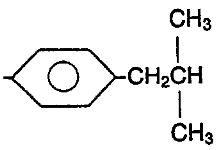 | $C_6H_5$ | 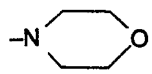 | H | | |
| 28 | 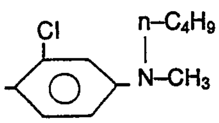 | 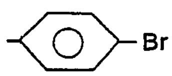 | 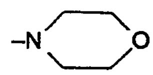 | H | | |
| 29 | 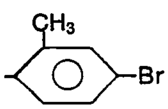 | 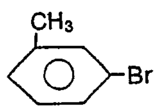 | 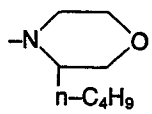 | H | | |
| 30 | 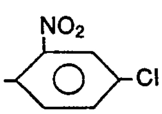 | 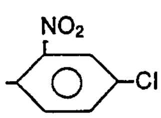 | 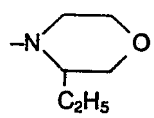 | H | | |
| 31 | 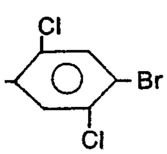 | 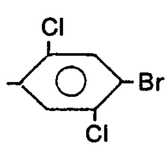 | 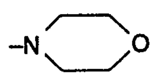 | $CH_3$ | | |
| 32 | 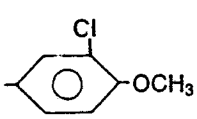 | 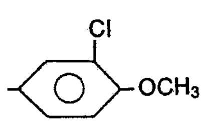 | 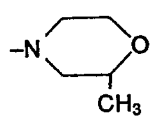 | n–$C_4H_9$ | | |

| Beispiel | A | B | Q | $R_1$ | Fp.(°C) | Rf |
|---|---|---|---|---|---|---|
| 33 | $-C_6H_5$ | 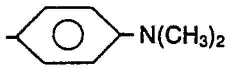 | 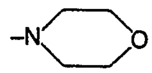 | H | 142 | |
| 34 | 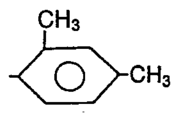 | $C_6H_5$ | 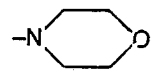 | H | 120 | |
| 35 | 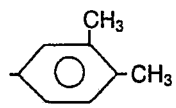 | $C_6H_5$ | 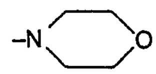 | H | | 0,55* |
| 36 | 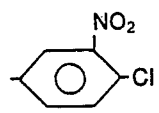 | $C_6H_5$ | 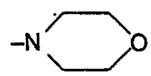 | H | | |
| 37 | 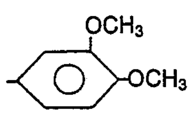 | 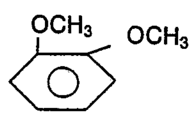 | 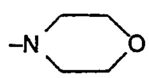 | H | | 0,34* |

*Rf-Werte: Laufmittel Toluol/Aceton 7:3. Es wurden DC-Platten Polygram der Fa. Macherey-Nagel (Art. Nr. 805 021) verwendet; 22 °C

Beispiel 38
3-(3,4-Dimethoxyphenyl)-5-phenylpenta-2,4-dien-1-carbonsäuremorpholid

a) 3,4-Dimethoxy-β-methylzimtsäureethylester

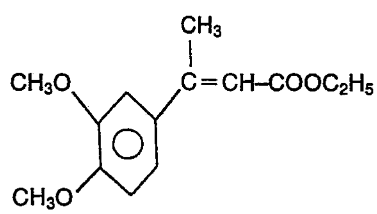

24,75 g (825 mmol) Natriumhydrid (mit 20% Paraffinöl) werden mit 300 ml abs. 1,2-Dimethoxyethan gerührt und unter Eiswasserkühlung 185 g (825 mmol) Triethylphosphonacetat zugetropft. Zu der klaren Lösung werden in einer Portion 135 g (750 mmol) 3,4-Dimethoxyacetophenon gegeben und 3 Stunden auf dem siedenden Wasserbad unter Rückfluss gerührt. Nach dem Stehen über Nacht wird das 1,2-Dimethoxyethan unter Vakuum abdestilliert und der Rückstand mit Toluol-Wasser geschüttelt. Die Toluollösung wird nochmals mit Wasser gewaschen, getrocknet und das Toluol unter Vakuum abdestilliert. Der Rückstand wird im Vakuum fraktioniert destilliert.

Ausbeute: 159 g (85% d.Th.)
Kp. 130 °C/0,04 mbar

b) 3-(3,4-Dimethoxyphenyl)-5-phenyl-penta-2,4-dien-1-carbonsäure

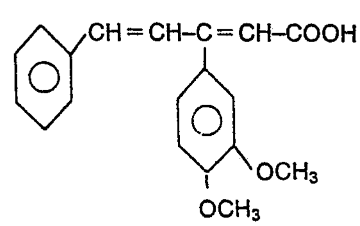

5,05 g (45 mmol) Kalium-tert.-butylat werden in 20 ml abs. Dimethylformamid gelöst und unter Rühren im Eisbad eine Lösung von 10,0 g (40 mmol) 3,4-Dimethoxy-β-methylzimtsäureethylester und 4,78 g (45 mmol) Benzaldehyd in 10 ml abs. Dimethylformamid zugetropft. Es wird 4 Stunden bei Raumtemperatur nachgerührt, mit Wasser versetzt und mit Salzsäure angesäuert. Die dabei erhaltene Schmiere wird mit heissem Wasser gerührt und portionsweise Natriumcarbonat bis zur Lösung zugegeben. Diese wässrige Lösung wird zweimal mit Toluol ausgeschüttelt. Beim Ansäuern mit Salzsäure fällt die Substanz aus und wird nach dem Waschen mit Wasser aus Toluol umkristallisiert.

Ausbeute: 9,7 g (78% d.Th.)
Fp. 164 °C.

c) 3-(3,4-Dimethoxyphenyl)-5-phenylpenta-2,4-dien-1-carbonsäuremorpholid

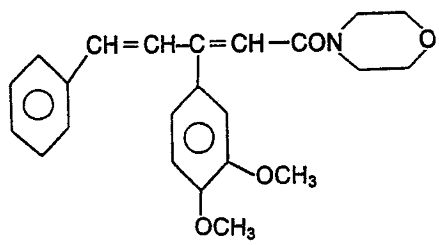

4,66 g (15 mmol) 3-(3,4-Dimethoxyphenyl)-5-phenylpenta-2,4-dien-1-carbonsäure werden in 30 ml abs. Tetrahydrofuran gelöst und portionsweise 3,24 g (20 mmol) 1,1′-Carbonyldiimidazol zugegeben. Nach Beendigung der $CO_2$-Entwicklung und klarer Lösung werden 1,74 g (20 mmol) Morpholin zugesetzt, 10 Minuten bei Raumtemperatur stehen gelassen und anschliessend 30 Minuten unter Rückfluss gekocht. Die Lösung wird unter Vakuum eingedampft und der Rückstand mit Toluol-Wasser geschüttelt. Die Toluollösung wird noch zweimal mit Wasser gewaschen, getrocknet und unter Vakuum eingedampft.

Ausbeute: 4,9 g (86% d.Th.) DC-reines Öl
Rf: 0,54 (Toluol-Aceton 70 : 30)

| | | |
|---|---|---|
| Analyse: | C ber. 72,80%, | gef. 73,12% |
| | H ber. 6,64%, | gef. 6,67% |
| | N ber. 3,69%, | gef. 3,64% |

Beispiel 39

a) 5-(4-Chlorphenyl)-3-(3,4-dimethoxyphenyl)-hexa-2,4-dien-1-carbonsäure

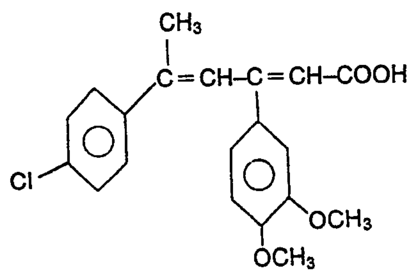

5,05 g (45 mmol) Kalium-tert.-butylat in 20 ml abs. Dimethylformamid gelöst und unter Rühren im Eisbad eine Lösung von 10,0 g (40 mmol) 3,4-Dimethoxy-β-methylzimtsäureethylester und 6,96 g (45 mmol) 4-Chloracetophenon in 10 ml abs. Dimethylformamid zugetropft. Es wird 4 Stunden bei Raumtemperatur nachgerührt, mit Wasser versetzt und mit Salzsäure angesäuert. Das dabei erhaltene zähe Öl wird mit heissem Wasser gerührt und portionsweise Natriumcarbonat bis zur Lösung zugegeben. Die wässrige Lösung wird einmal mit Toluol gewaschen. Beim Ansäuern mit Salzsäure fällt die Substanz wiederum ölig an. Diese wird mit Toluol extrahiert, der Extrakt zweimal mit Wasser gewaschen, getrocknet und das Toluol unter Vakuum abdestilliert.

Ausbeute: 11,9 g (83% d.Th., Öl)

b) 5-(4-Chlorphenyl)-3-(3,4-dimethoxyphenyl)-hexan-2,4-dien-1-carbonsäuremorpholid

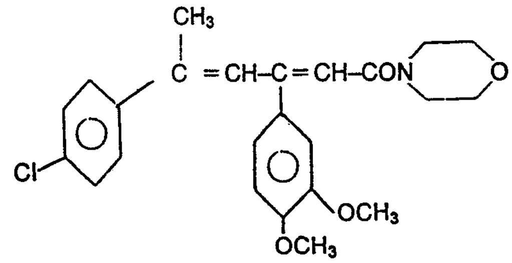

5,38 g (15 mmol) 5-(4-Chlorphenyl)-3-(3,4-dimethoxyphenyl)-hexa-2,4-dien-1-carbonsäure werden in 30 ml abs. Tetrahydrofuran gelöst und portionsweise 3,24 g (20 mmol) 1,1′-Carbonyldiimidazol zugegeben. Nach Beendigung der $CO_2$-Entwicklung werden 1,74 g (20 mmol) Morpholin zugesetzt, 10 Minuten bei Raumtemperatur stehen gelassen und anschliessend 30 Minuten unter Rückfluss gekocht. Die Lösung wird unter Vakuum eingedampft und der Rückstand mit Toluol-Wasser geschüttelt. Die Toluolphase wird nochmals mit Wasser gewaschen, getrocknet und unter Vakuum auf ca. 20 ml eingeengt. Diese Lösung wird über eine mit 30 g mit Toluol angerührtem Kieselgel beschickte Chromatographiesäule gereinigt. Eluiert wird zunächst mit Toluol, dann mit Toluol-Aceton-Gemisch 90 : 10. Die Fraktionen mit der Substanz vom Rf 0,47 (Toluol-Aceton 70 : 30 auf Kieselgel-Platte) werden gesammelt und unter Vakuum eingedampft.

Ausbeute: 4,7 g (73% d.Th.), Öl

| | | |
|---|---|---|
| Analyse: | C ber. 67,36%, | gef. 67,38% |
| | H ber. 6,12%, | gef. 6,22% |
| | N ber. 3,27%, | gef. 3,13% |

Beispiel 40

a) 3-(3,4-Dimethoxyphenyl)-2-methylcrotonsäureethylester

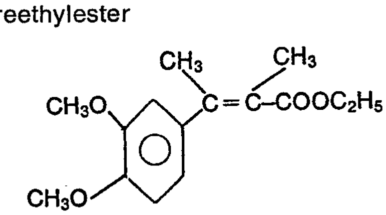

3,3 g (110 mmol) Natriumhydrid (mit 20% Paraffinöl) werden mit 40 ml abs. 1,2-Dimethoxyethan gerührt und unter Eiswasserkühlung 26,2 g (110 mmol) Triethyl-2-phosphonpropionat zugetropft. Nach erfolgter klarer Lösung werden 18 g (100 mmol) 3,4-Dimethoxyacetophenon zugegeben und 3 Stunden unter Rühren unter Rückfluss gekocht. Nach dem Stehen über Nacht wird unter Vakuum das 1,2-Dimethoxyethan abdestilliert und

der Rückstand mit Toluol-Wasser geschüttelt. Die Toluolphase wird nochmals mit Wasser gewaschen, getrocknet und das Toluol unter Vakuum abdestilliert. Der Rückstand wird im Vakuum fraktioniert destilliert.

Ausbeute: 19,8 g (81% d.Th.)
Kp. 145 °C/0,4 mbar

b) 5-(4-Chlorphenyl)-3-(3,4-dimethoxyphenyl)-2-methylpenta-2,4-dien-1-carbonsäure

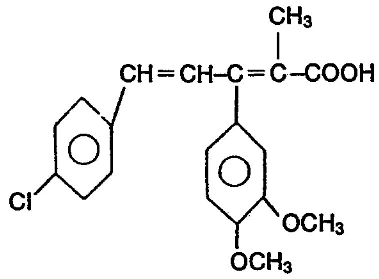

6,17 g (55 mmol) Kalium-tert.-butylat werden in 20 ml abs. Dimethylformamid gelöst und unter Rühren im Eisbad eine Lösung von 13,2 g (50 mmol) 3-(3,4-Dimethoxyphenyl)-2-methylcrotonsäureethylester und 7,73 g (55 mmol) 4-Chlorbenzaldehyd in 20 ml abs. Dimethylformamid zugetropft. Es wird 6 Stunden bei Raumtemperatur nachgerührt, mit Wasser versetzt und mit Salzsäure angesäuert. Die dabei erhaltene Schmiere wird mit heissem Wasser gerührt und portionsweise Natriumcarbonat bis zur Lösung zugegeben. Diese wässrige Lösung wird einmal mit Toluol ausgeschüttelt. Das beim Ansäuern mit Salzsäure erhaltene Öl wird aus Toluol-Petrolether umkristallisiert.

Ausbeute: 11 g (61% d.Th.);
Fp. 164 °C

c) 5-(4-Chlorphenyl-3-(3,4-dimethoxyphenyl)-2-methylpenta-2,4-dien-1-carbonsäuremorpholid

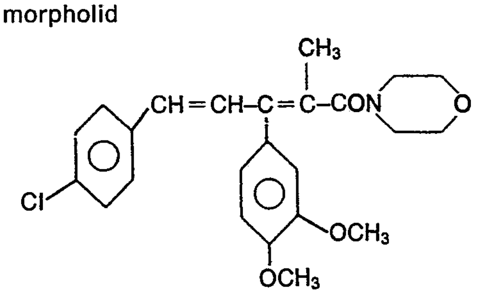

5,38 g (15 mmol) 5-(4-Chlorphenyl-3-(3,4-dimethoxyphenyl)-2-methylpenta-2,4-dien-1-carbonsäure werden in 30 ml abs. Tetrahydrofuran gelöst und portionsweise mit 3,24 g (20 mmol) 1,1'-Carbonyldiimidazol versetzt. Nach Beendigung der $CO_2$-Entwicklung werden 1,74 g (20 mmol) Morpholin zugesetzt, 10 Minuten bei Raumtemperatur stehen gelassen und anschliessend 2 Stunden unter Rückfluss gekocht. Die Lösung wird unter Vakuum eingedampft und der Rückstand mit Toluol-Wasser geschüttelt. Die Toluolphase wird nochmals mit Wasser gewaschen, getrocknet und unter Vakuum auf ca. 20 ml eingeengt. Diese Lösung wird über eine mit 30 g mit Toluol angerührtem Kieselgel beschickte Chromatographiersäule gereinigt. Eluiert wird zunächst mit Toluol, danach mit Toluol-Aceton-Gemisch 90 : 10. Die Fraktionen mit der Substanz vom Rf 0,52 (Toluol-Aceton 70 : 30 auf Kieselgel-Platte) werden gesammelt und unter Vakuum eingedampft.

Ausbeute: 4,95 g (77% d.Th.); Öl

| | | |
|---|---|---|
| Analyse: | C ber 67,36%, | gef. 67,61% |
| | H ber. 6,13%, | gef. 6,10% |
| | N ber. 3,27%, | gef. 3,36% |

Beispiel 41
5-(4-Chlorphenyl)-3-(3,4-dimethoxyphenyl)-penta-2,4-dien-1-carbonsäuremorpholid

a) 3,4-Dimethoxyacetophenon

Zu 343,5 g (2,5 mol) Veratrol, suspendiert in 1400 ml Ethylenchlorid, lässt man 215,9 g (2,75 mol) Acetylchlorid zulaufen. Man rührt 30 Minuten bei Raumtemperatur, kühlt mit Eis/Kochsalz und gibt 366,6 g (2,75 mol) $AlCl_3$ zu. Es wird 3 Stunden gerührt, wobei die Temperatur unter 15 °C gehalten wird. Nach Aufarbeitung und Destillation am Rotationsverdampfer erhält man 419,1 g (93% d.Th.) eines Öls, Kp. 125–127 °C/0,5 mbar. Aus $CCl_4$/Petrolether erhält man eine Substanz mit dem Fp. 48–50 °C.

b) 3,4-Dimethoxy-β-methylzimtsäureethylester

Zu 291,7 g (2,6 mol) Kalium-tert.-butylat, suspendiert in 1,7 Ltr. abs. 1,2-Dimethoxyethan lässt man 576,2 g (2,57 mol) Triethylphosphonacetat zulaufen, rührt 15 Minuten und lässt dann 463 g (2,57 mol) öliges 3,4-Dimethoxyacetophenon zulaufen.

Die Mischung wird 7 Stunden bei ca. 60 °C gerührt. Das Lösungsmittel wird dann am Rotationsverdampfer entfernt, der Rückstand mit Wasser versetzt und ausgeethert. Aus der Etherphase isoliert man den 3,4-Dimethoxy-β-methylzimtsäureethylester in einer Ausbeute von 558 g (87% d.Th.);
Kp. 173 °C/0,17 mbar

c) 3-(3,4-Dimethoxyphenyl)-5-(4-chlorphenyl)-penta-2,4-dien-1-carbonsäure

28,1 g (0,25 mol) Kalium-tert.-butylat in 75 ml Dimethylformamid werden vorgelegt. Unter Kühlung werden bei 10–15 °C 30,7 g (0,22 mol) 4-Chlorbenzaldehyl und 50 g (0,20 mol) 3,4-Dimethoxy-β-methylzimtsäureethylester in 125 ml Dimethylformamid innerhalb von 10 Minuten dazugegeben. Die Mischung wird 45 Minuten ohne Kühlung gerührt, mit 350 ml Wasser versetzt und mit 50 ml 6 N Salzsäure angesäuert. Das ausgefallene Produkt wird abgesaugt und aus Dioxan umkristallisiert.

Ausbeute 57,6 g (84% d.Th.);
Fp. 226–227 °C.

d) 3-(3,4-Dimethoxyphenyl)-5-(4-chlorphenyl)-penta-2,4-dien-1-carbonsäuremorpholid

Zu 4,52 g (52 mmol) Morpholin in 15 ml Pyridin tropft man unter Kühlung bei −5 °C bis 0 °C in 10 Minuten 2,38 g (17,3 mmol) Phosphortrichlorid. Nach halbstündigem Nachreagieren bei Raumtemperatur werden 12,1 g (35 mmol) der nach c)

erhaltenen Säure, aufgeschlämmt in 35 ml Pyridin, auf einmal zugegeben. Man lässt 3 Stunden bei Raumtemperatur stehen und rührt dann noch $^1/_2$ Stunde bei 40 °C. Die Mischung wird dann mit Eis und konzentrierter Salzsäure versetzt bis zur stark sauren Reaktion. Durch Zugabe von Essigester wird das Reaktionsprodukt ausgefällt. Es wird abgesaugt, mit Wasser und Essigester gewaschen und aus Isopropanol/Wasser umkristallisiert.

Ausbeute 12,3 g (85% d.Th.);
Fp. 125–126 °C.

Beispiel 42

3-(3,4-Methylendioxyphenyl)-5-(4-chlorphenyl)-penta-2,4-dien-1-carbonsäureisopropylamid

a) 3-(3,4-Methylendioxyphenyl)-5-(4-chlorpenyl)-penta-2,4-dien-1-carbonsäure

Zu einer Aufschlämmung von 35 g (0,25 mol) Kalium-tert.-butylat in 80 ml Dimethylformamid werden unter Kühlung 29 g (0,26 mol) 4-Chlorbenzaldehyd und 55 g (0,235 mol) 3,4-Methylen-dioxy-β-methylzimtsäureethylester in 160 ml Dimethylformamid so innerhalb von 10 Minuten zugegeben, dass die Temperatur 20 °C nicht übersteigt. Nach einstündigem Rühren ohne Kühlung werden Wasser und Salzsäure (bis zur sauren Reaktion) zugegeben und das ausfallende Produkt abgesaugt.

Es lässt sich aus viel Isopropanol umkristallisieren.

Ausbeute: 67,8 g (87% d.Th.);
Fp. 205–206 °C.

b) 3-(3,4-Methylendioxyphenyl)-5-(4-chlorphenyl)penta-2,4-dien-1-carbonsäureisopropylamid

Aus 5,9 g (0,10 mol) Isopropylamin in 25 ml Pyridin und 4,6 g (0,034 mol) Phosphortrichlorid stellt man in üblicher Weise die Phosphazoverbindung her. 22,0 g (0,067 mol) der nach a) erhaltenen Säure werden zugegeben, 4 Stunden gerührt und über Nacht stehen gelassen. Nach Zugabe von Eis und Ansäuern mit Salzsäure wird mit Essigester extrahiert, der Essigesterextrakt mit Wasser und verdünnter Natronlauge gewaschen, getrocknet und eingeengt. Der Rückstand kann aus Benzol/Petrolether umkristallisiert werden.

Ausbeute 10,8 g (43,5% d.Th.);
Fp. 125–127 °C.

Die Rf-Werte in der nachstehenden Tabelle wurden auf DC-Platten der Firma Macherey-Nagel (Art.-Nr. 805 021) bestimmt. Laufmittel Toluol/Aceton 7 : 3 22 °C.

Entsprechend den Beispielen werden auch die unten aufgeführten Verbindungen erhalten:

Tabelle II

Verbindungen der Formel

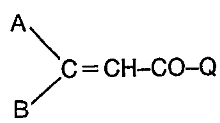

| Beispiel | Q | A | B | Fp./Rf |
|---|---|---|---|---|
| 43 | $N(CH_3)_2$ | 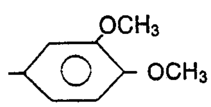 | 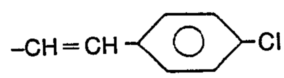 | Rf:0,53 |
| 44 | $NHCH(CH_3)_2$ | " | " | 146 °C |
| 45 | $N(CH_3)$<br>\|<br>$CH_2–C_6H_5$ | " | " | Rf: 0,71 |
| 46 | $N(C_2H_5)_2$ | " | " | Rf: 0,63 |
| 47 | $N(CH_3)$<br>\|<br>$n–C_3H_7$ | " | " | Rf: 0,69 |
| 48 | 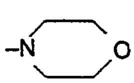 | " | 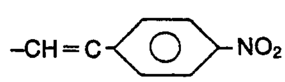 | |

| Beispiel | Q | A | B | Fp./Rf |
|---|---|---|---|---|
| 49 | 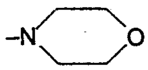 | 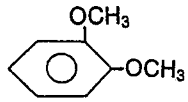 | 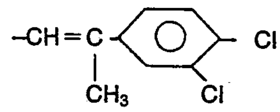 | |
| 50 | " | " | 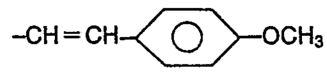 | Rf: 0,59 |
| 51 | " | " | 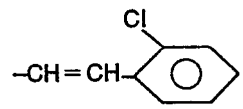 | Rf:0,53 |
| 52 | " | " | 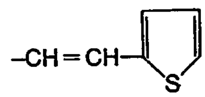 | |
| 53 | " | " | 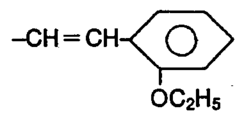 | 123 °C |
| 54 | " | " | 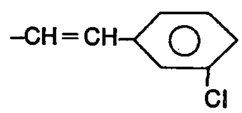 | 130 °C |
| 55 | " | " | 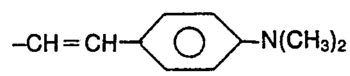 | Rf: 0,36 |
| 56 | " | " | 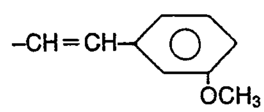 | Rf: 0,51 |
| 57 | " | " | 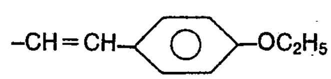 | Rf: 0,52 |
| 58 | " | " | 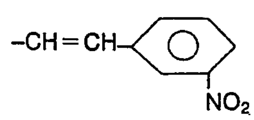 | 152 °C |
| 59 | " | " | 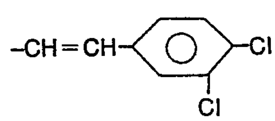 | Rf: 0,54 |
| 60 | " | „ | 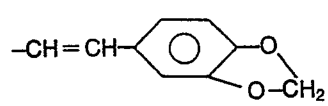 | 158 °C |

| Beispiel | Q | A | B | Fp./Rf |
|---|---|---|---|---|
| 61 | 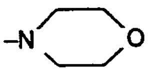 | 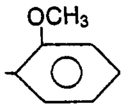 | –CH=CH– 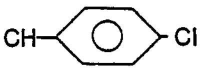 | 145 °C |
| 62 | " | 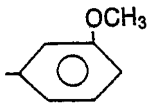 | " | Rf: 0,62 |
| 63 | " | 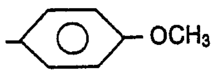 | " | 122 °C |
| 64 | " | 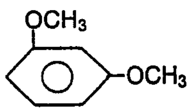 | $–CH=CH–C_6H_5$ | 149 °C |
| 65 | " | 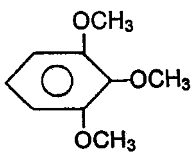 | –CH=CH– 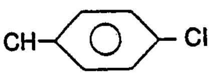 | 80 °C |
| 66 | " | 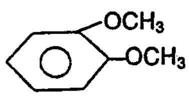 | –CH=CH– 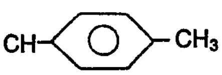 | |
| 67 | " | 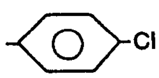 | –CH=CH– 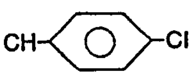 | 181 °C |
| 68 | " | " | –CH=CH– 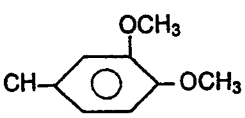 | Rf: 0,51 |
| 69 | " | 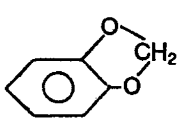 | –CH=CH– 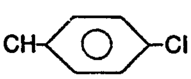 | 102 °C |
| 70 | $–NHCHCCH_3)_2$ | " | " | 132 °C |
| 71 | 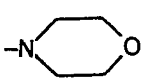 | 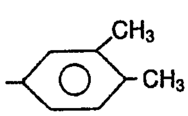 | –CH=CH– 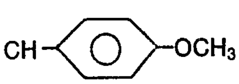 | Rf: 0,62 |

| Beispiel | Q | A | B | Fp./Rf |
|---|---|---|---|---|
| 72 | 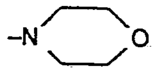 | 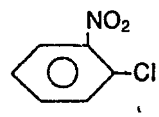 | $-CH=CH-C_5H_5$ | 151 °C |
| 73 | 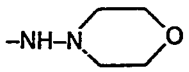 | 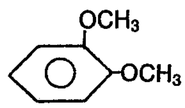 | $-CH=CH$ 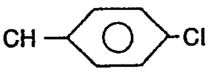 | 138 °C |
| 74 | 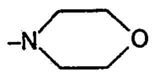 | " | $-(CH=CH)_2-C_6H_5$ | 80 °C |
| 75 | " | " | 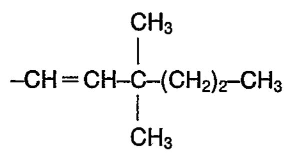 | Rf: 0,46 |
| 76 | " | " | $-CH=CH$ 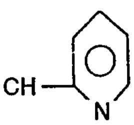 | Rf: 0,50 Toluol/ Ethanol 8:2 |
| 77 | " | " | $-CH=CH$ 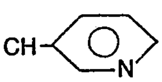 | 165 °C |
| 78 | " | " | $-CH=CH$ 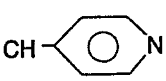 | Rf: 0,13 |
| 79 | " | " | $-CH=CH$ 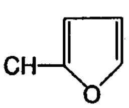 | Rf: 0,46 |
| 80 | $-NHCH(CH_3)_2$ | 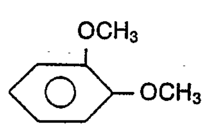 | $-CH=C(CH_3)_2$ | 112 °C |
| 81 | " | " | $-CH=CH-CH(CH_3)_2$ | 98 °C |
| 82 | " | " | $-CH=$ 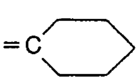 | 62 °C |

| Beispiel | Q | A | B | Fp./Rf |
|---|---|---|---|---|
| 83 | 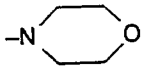 | 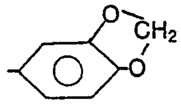 | –CH=CH– 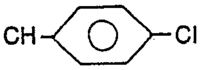 | 102 °C |
| 84 | " | $-C_6H_5$ | " | 127 °C |
| 85 | $-NH-CH(CH_3)_2$ | 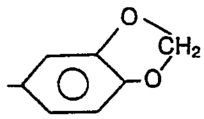 | –CH=CH– 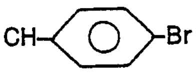 | 126 °C |
| 86 | 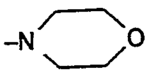 | 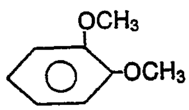 | $-CH=C(CH_3)_2$ | Öl |
| 87 | " | 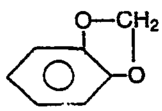 | $-CH=CH-CH(CH_3)_2$ | 76 °C |
| 88 | $-NH-CH(CH_3)_2$ | " | $-CH=C(CH_3)_2$ | 133 °C |
| 89 | " | " | $-C(CH_3)=C(CH_3)_2$ | 97 °C |
| 90 | 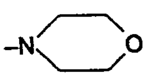 | 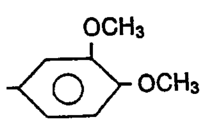 | –CH= 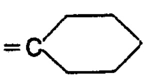 | Öl |
| 91 | " | $-C_6H_5$ | –CH=CH– 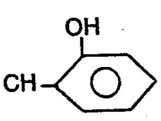 | 178 °C |
| 92 | " | 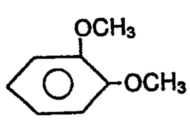 | $-CH=CH-CH(CH_3)_2$ | Öl |

Beispiel 93

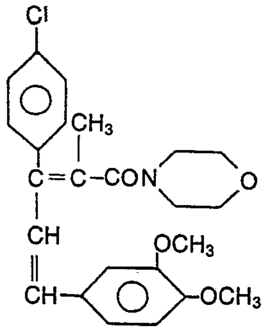

Fp. 157 °C

Beispiel 94

2-Cyan-3-(3,4-dimethoxyphenyl)-3-phenylacrylsäuremorpholid

12,1 g (50 mMol) 3,4-Dimethoxybenzophenon, 8,48 g (55 mMol) Cyanacetmorpholid, 10 g Eisessig, 4 g Ammonacetat und 100 ml Benzol werden unter Rühren am Wasserabscheider erhitzt. Nach 5, 10 und 15 Stunden werden jeweils 4 g Ammonacetat und 5 g Eisessig zugesetzt. Insgesamt wird 20 Stunden erhitzt, anschliessend Benzol und Eisessig am Rotationsverdampfer abdestilliert. Der Rückstand wird mit Toluol/Wasser geschüttelt, die organische Phase nochmals mit Wasser gewaschen, getrocknet und im Vakuum eingeengt. Dieses Konzentrat wird über eine Kieselgel-Säule gereinigt, wobei zunächst mit Toluol, dann mit Toluol-Aceton-Gemisch 80 : 20 eluiert wird. Die Fraktionen mit der Substanz vom Rf-Wert 0,56 (Toluol-Aceton 70 : 30) werden im Vakuum vollständig eingedampft.

Ausbeute: 9,7 g (51% d.Th); zähflüssiges gelbes Öl

E/Z-Verhältnis 50 : 50.

| | | |
|---|---|---|
| Analyse: ber. | C 69,83%, | gef.: 69,64% |
| | H 6,36%, | 6,07% |
| | N 7,40%, | 7,66% |

Beispiel 95

3-(3-Acetamino-4-methoxyphenyl)-3-phenylacrylsäuremorpholid

3,38 g (10 mMol) 3-(3-Amino-4-methoxyphenyl)-3-phenylacrylsäuremorpholid werden in 20 ml Acetanhydrid gelöst und 4 Stunden auf dem siedenden Wasserbad erwärmt. Die Lösung wird im Vakuum eingedampft und der Rückstand aus Essigester-Petroläther umkristallisiert.

Ausbeute: 3,1 g (81,5% d.Th.); Fp. 97 °C

Rf-Wert 0,50 (Toluol-Aceton 30 : 70)

| | | |
|---|---|---|
| Analyse: ber.: | C 69,46%, | gef.: 69,67% |
| | H 6,36%, | 6,48% |
| | N 7,37%, | 7,24% |

Beispiel 96

3-(3-Amino-4-mentoxyphenyl)-3-phenylacrylsäuremorpholid

7,37 g (20 mMol) 3-C4-Methoxy-3-nitrophenyl)-3-phenylacrylsäuremorpholid werden in 100 ml Ethanol gelöst, 100 ml Wasser zugesetzt, auf 50 °C erwärmt und unter Rühren 14,7 g (70 mMol) Natriumdithionit zugegeben. Dann wird eine Stunde unter Rückfluss gekocht, Ethanol im Vakuum abdestilliert und Wasser zugegeben. Die dabei erhaltene braune Schmiere wird in Toluol gelöst und über eine Kieselgel-Säule gereinigt. Eluiert wird zunächst mit Toluol, dann mit Toluol-Aceton-Gemisch 80 : 20. Die Fraktionen mit der Substanz vom Rf-Wert 0,26 (Toluol-Aceton 70 : 30) werden im Vakuum eingedampft.

Ausbeute: 4,4 g (65% d.Th.); zähes Öl

E/Z-Verhältnis 30 : 70

| | | |
|---|---|---|
| Analyse: ber.: | C 70,98%, | gef.: 71,06% |
| | H 6,55% | 6,68% |
| | N 8,28% | 8,02% |

Beispiel 97

3-(4-Methoxy-3-methylphenyl)-3-phenylthioacrylsäuremorpholid

6,75 g (20 mMol) 3-C4-Methoxy-3-methylphenyl)-3-phenylacrylsäuremorpholid und 2,22 g (10 mMol) fein gemörsertes Phsphorpentasulfid werden mit 50 ml abs. Xylol 3 Stunden unter Rückfluss gekocht. Nach dem Erkalten wird von Unlöslichem abfiltriert, das Filtrat mit Wasser gewaschen, getrocknet und unter Vakuum eingeengt. Dieses Konzentrat wird über eine Säule mit Kieselgel gereinigt. Eluiert wird zunächst mit Toluol, anschliessend mit Toluol-Diispropylether-Gemisch 90 : 10, dann 80 : 20. Die Fraktionen mit der Substanz vom Rf-Wert 0,59 (Toluol-Diisopropylether 1 : 1) werden unter Vakuum eingedampft, das dabei erhaltene gelbe Öl unter Erwärmen in Cyclohexan gelöst. Beim Abkühlen kristallisiert die Substanz aus.

Ausbeute: 4,0 g (56,5% d.Th.); Fp. 125–127 °C

| | | |
|---|---|---|
| Analyse: ber.: | C 71,35%, | gef. 71,52% |
| | H 6,56% | 6,59% |
| | N 3,96% | 4,07% |
| | S 9,07% | 8,98% |

In den folgenden Tabellen sind weitere erfindungsgemäss herstellbare Verbindungen aufgeführt.

Tabelle III

Verbindungen der Formel

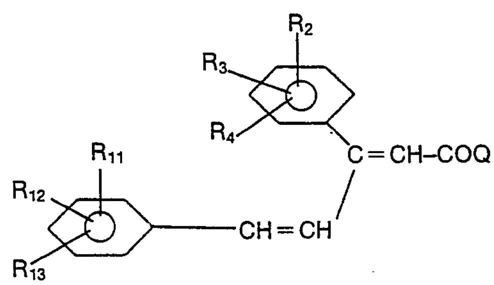

Rf-Werte:
In Toluol/Aceton
70:30, ca. 22°C

| Beispiel | $R_2/R_3/R_4$ | $R_{11}/R_{12}/R_{13}$ | Q | Fp. oder Rf |
|---|---|---|---|---|
| 98 | 3,4–$(CH_3O)_2$ | 4–Cl | $N(CH_3)C_4H_9$ | 0,70 |
| 99 | 3,4–$(CH_3O)_2$ | 4–Cl | $N(CH_3)CH(CH_3)–C_2H_5$ | 0,61 |
| 100 | 3–$NO_2$/4–Cl | H | 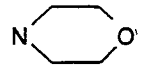 | 151 °C |
| 101 | 2,3,4–$(CH_3O)_3$ | H | " | 125 °C |
| 102 | 2,3,4–$(CH_3O)_3$ | 4–Cl | " | 127 °C |
| 103 | 3,4–(O–$CH_2CH_2$–O) | H | " | 0,56 |
| 104 | 3,4–(O–$CH_2CH_2$–O) | 4–Cl | " | 75–80 °C |
| 105 | 3–$CH_3$/4–$CH_3O$ | H | " | 137 °C |
| 106 | 3–$CH_3$/4–$CH_3O$ | 4–Cl | " | 151 °C |
| 107 | 3,4–($OCH_2O$) | H | " | 112 °C |
| 108 | 3–F/4–$CH_3O$ | H | " | 138 °C |
| 109 | 3–Cl/4–$CH_3O$ | H | " | 153 °C |
| 110 | 3–Br/4–$CH_3O$ | H | " | 160 °C |
| 111 | 3–Br/4–$CH_3O$ | 4–Cl | " | 150 °C |
| 112 | 2,5–$(CH_3O)_2$ | H | " | 0,58 |
| 113 | 2,5–$(CH_3O)_2$ | 4–Cl | " | 124 °C |
| 114 | 3–$CH_30$/4–$C_2H_5O$ | H | " | 0,63 |
| 115 | 3–$CH_30$/4–$C_2H_5O$ | 4–Cl | " | 0,60 |
| 116 | 3–$CH_3O$/4–$(CH_3)_2CHO$ | H | " | 117 °C |

| Beispiel | $R_2/R_3/R_4$ | $R_{11}/R_{12}/R_{13}$ | Q | Fp. oder Rf |
|---|---|---|---|---|
| 117 | $3\text{-}CH_3O/4\text{-}CH_2{=}CH\text{-}CH_2O$ | H | 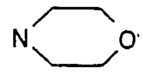 | 143 °C |
| 118 | $4\text{-}C_6H_5$ | 4-Cl | " | 166 °C |
| 119 | $3{,}5\text{-}Cl_2/4\text{-}NH_2$ | H | " | 163 °C |

Analysen der öligen Produkte der Tabelle II

| Beispiel | ber. % C | gef. % C | ber. % H | gef. % H | ber. % N | gef. % N |
|---|---|---|---|---|---|---|
| 98 | 69,64 | 69,67 | 6,82 | 6,73 | 3,38 | 3,17 |
| 99 | 69,64 | 69,96 | 6,82 | 6,76 | 3,38 | 3,68 |
| 103 | 73,19 | 73,41 | 6,14 | 6,26 | 3,71 | 3,41 |
| 112 | 72,80 | 73,13 | 6,64 | 6,77 | 3,69 | 3,65 |
| 114 | 73,26 | 73,33 | 6,92 | 6,95 | 3,56 | 3,57 |
| 115 | 67,36 | 67,68 | 6,12 | 6,39 | 3,27 | 2,96 |

Tabelle III

Verbindungen der Formel

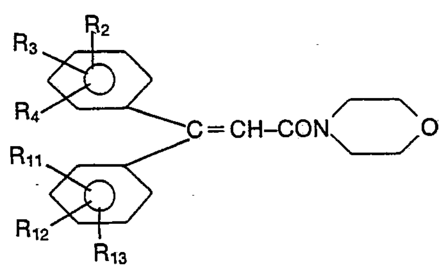

Rf-Werte:
In Toluol/Aceton
70:30, ca 22 °C

| Beispiel | $R_2/R_3/R_4$ | $R_{11}/R_{12}R_{13}$ | Fp. oder Rf | Bemerkungen |
|---|---|---|---|---|
| 120 | $4\text{-}N(CH_3)_2$ | 4-Cl | | |
| 121 | $2{,}4\text{-}(CH_3)_2$ | 4-Cl | | |
| 122 | $3{,}4\text{-}(CH_3)_2$ | 4-Cl | | |
| 123 | $3\text{-}NO_2/4\text{-}CH_3O$ | H | 124 °C | |
| 124 | $3{,}4\text{-}(CH_3O)_2$ | $3{,}4\text{-}Cl_2$ | | |
| 125 | $3{,}4\text{-}(CH_3O)2$ | H | 100 °C | Isomerengemisch |
| 126 | $3{,}4\text{-}(CH_3O)2$ | H | 135–137 °C | Z-Isomeres |
| 127 | $3\text{-}CH_3/4\text{-}CH_3O$ | H | 106–110 °C | Isomerengemisch |
| 128 | $3\text{-}CH_3/4\text{-}CH_3O$ | H | 125–127 °C | E-Isomeres |
| 129 | $3\text{-}CH_3/4\text{-}CH_3O$ | H | 115–118 °C | Z-Isomeres |
| 130 | $3{,}4\text{-}Cl_2$ | H | 0,5 + 0,56 | |
| 131 | $3{,}4\text{-}(CH_3O)_2$ | $3{,}4\text{-}(CH_3O)_2$ | 70 | |
| 132 | $3{,}4\text{-}(CH_3O)_2$ | 4-Cl | 135–137 °C | |
| 133 | $3{,}5\text{-}(CH_3)_2/4\text{-}CH_3O$ | H | 150 °C | |
| 134 | $3{,}4\text{-}(C_2H_5)_2$ | H | 0,58 | |
| 135 | $3{,}4\text{-}(CH_3O)_2$ | 3-Cl | Harz | |
| 136 | $3{,}4\text{-}(CH_3O)_2$ | 2-Cl | 126–129 °C | |
| 137 | $3\text{-}Br/4\text{-}CH_3O$ | $4\text{-}CH_3O$ | 0,48 | Isomerengemisch |

| Beispiel | $R_2/R_3/R_4$ | $R_{11}/R_{12}R_{13}$ | Fp. oder Rf | Bemerkungen |
|---|---|---|---|---|
| 138 | 3–Br/4–$CH_3O$ | 4–$CH_3O$ | 161 °C | Z-Isomeres |
| 139 | 3–$C_2H_5$/4–$CH_3O$ | H | 93 °C | |
| 140 | 3–n–$C_3H_7$/4–$CH_3O_3$ | H | 0,60 | |
| 141 | 3–$CH_3O$/4–$CH_3$ | H | 0,53 | E-Isomeres |
| 142 | 3–$CH_3O$/4–$CH_3$ | H | 119 °C | E-Isomeres |
| 143 | 3–Cl/4–$CH_3O$ | 3–Cl, 4–$CH_3O$ | 0,50 | |
| 144 | 3–$CH_3$/4–OH | H | 0,42 | |
| 145 | 3–$NH_2$/4–$CH_3O$ | 4–Cl | | |
| 146 | 3,4–$(CH_3O)_2$ | 2–F | 118–127 °C | |
| 147 | 2,3–$(CH_3)_2$/4–$CH_3O$ | H | 171–174 °C | |
| 148 | 3–$CH(CH_3)_2$/4–$CH_3O$ | H | 134–136 °C | |
| 149 | 3–$CH_3$/4–$NO_2$ | 4–$CH_3O$ | 0,42 | |
| 150 | 3–$NH_2$/4–Cl | H | 65–67 °C | |
| 151 | 3–$CH_3$/4–$CH_3O$ | 3–$CH_3$/4–$CH_3O$ | 0,47 | |
| 152 | 3–$NHCOCH_3$/4–$CH_3O$ | 4–Cl | | |

Analysen der öligen Produkte der Tabelle III

| Beispiel | ber. % C | gef. % C | ber. % H | gef. % H | ber. % N | gef. % N |
|---|---|---|---|---|---|---|
| 122 | 70,87 | 70,71 | 6,23 | 6,10 | 3,94 | 4,05 |
| 124 | 59,72 | 59,87 | 5,01 | 5,21 | 3,32 | 3,17 |
| 130 | 62,99 | 63,20 | 4,73 | 4,67 | 3,87 | 3,70 |
| 134 | 79,05 | 78,96 | 7,79 | 7,78 | 4.01 | 3,86 |
| 135 | 65,03 | 64,74 | 5,68 | 5,77 | 3,61 | 3,60 |
| 137 | 58,34 | 58,67 | 5,13 | 5,25 | 3,24 | 3,22 |
| 140 | 75,59 | 75,89 | 7,45 | 7,69 | 3,83 | 3,69 |
| 141 | 74,75 | 74,55 | 6,87 | 7,01 | 4.15 | 4,17 |
| 143 | 59,72 | 59,91 | 4,98 | 5,12 | 3,32 | 3,18 |
| 144 | 74,30 | 74,61 | 6,50 | 6,73 | 4,33 | 4,01 |
| 145 | 70,98 | 71,06 | 6,55 | 6,68 | 8,28 | 8,16 |
| 148 | 75,62 | 75,61 | 7,40 | 7,35 | 3,84 | 4,03 |
| 149 | 65,97 | 65,73 | 5,76 | 5,91 | 7,33 | 7,19 |
| 151 | 72,42 | 72,54 | 7,13 | 7,26 | 3,67 | 3,50 |

Tabelle IV

Verbindungen der Formel

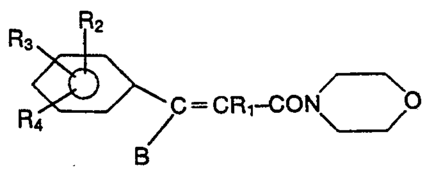

| Beispiel | $R_1$ | $R_2/R_3/R_4$ | B | Fp. oder Rf |
|---|---|---|---|---|
| 153 | H | 3,4–$(CH_3O)_2$ | 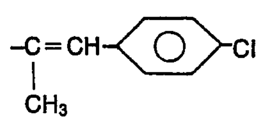 | 127 °C |
| 154 | H | 3,4–$(CH_3O)_2$ | 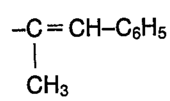 | 145 °C |
| 155 | Br | 3–Br/4–$CH_3O$ | 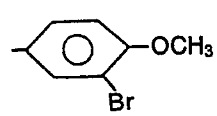 | 93 °C |
| 156 | H | 3,4–$(CH_3O)_2$ | 2-Naphthyl | Harz/Analyse |

| Beispiel | $R_1$ | $R_2/R_3/R_4$ | B | Fp. oder Rf |
|---|---|---|---|---|
| 157 | H | $3,4-(CH_3O)_2$ | 1-Naphthyl | 0,43 |
| 158 | H | $3,4-(CH_3O)_2$ | 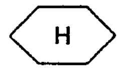 | 117–120 |
| 159 | H | $3,4-(CH_3O)_2$ | $CH_2C_6H_5$ | 0,46 + 0,56 |
| 160 | H | $3,4-(CH_3O)_2$ | $CH(CH_3)_2$ | Öl |
| 161 | H | H | 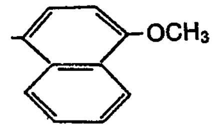 | 228–231 °C |
| 162 | H | $3,4-(CH_3O)_2$ | 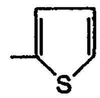 | 130–132 °C |
| 163 | $CH_3$ | $3,4-(CH_3O)_2$ | $C_6H_5$ | 0,50 + 0,60 |
| 164 | CN | $3,4-(CH_3O)_2$ | $C_6H_5$ | |

Analysen zu Tabelle IV

| Beispiel | ber. % Cl | gef. % C | ber. % H | gef. % H | ber. % N | gef. % N |
|---|---|---|---|---|---|---|
| 156 | 74,44 | 74,69 | 6,20 | 6,48 | 3,47 | 3,29 |
| 157 | 74,44 | 74,73 | 6,20 | 6,40 | 3,47 | 3,32 |
| 159 | 71,93 | 72,05 | 6,81 | 6,92 | 3,81 | 3,77 |
| 160 | 67,71 | 67,48 | 7,84 | 7,79 | 4,39 | 4,33 |
| 163 | 71,91 | 72,20 | 6,86 | 6,98 | 3,81 | 3,74 |

**Patentansprüche**

1. Verbindungen der Formel

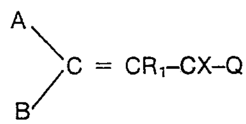 (I),

in der
A für die Gruppe

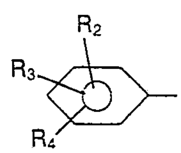 (II),

B für die Gruppe

$$Y-(CR_5=CR_6)_k- \quad \text{(III)},$$

Q für eine der Gruppen

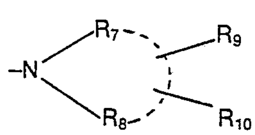 (IV)

und

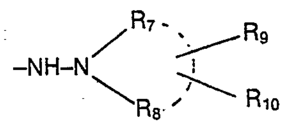 (V)

X für O, S oder NH,

y für $C_3$–$C_{10}$-Alkyl sowie zusätzlich für $C_1$–$C_2$-Alkyl, wenn k gleich 1 oder 2 ist, oder für substituiertes $C_1$–$C_{10}$-Alkyl oder für einen gegebenenfalls substituierten Rest aus der Gruppe $C_3$–$C_7$-Alkenyl, $C_3$–$C_7$-Cycloalkyl, $C_5$–$C_7$-Cycloalkenyl, Pyridyl, Furyl, Thienyl, α- und β-Naphthyl, wobei die Substituenten Halogen, Nitro, Amino, gegebenenfalls ein- oder mehrfach halogensubstituierte $C_1$–$C_4$-Alkyl- und Alkoxygruppen, $-NH(C_1–C_4\text{-Alkyl})$, $-N(C_1–C_4\text{-Alkyl})_2$, Phenoxy, Phenylthio oder $C_1$–$C_4$-Alkylthio sind; oder zusammen mit der Gruppe $CR_5$ für einen 5- bis 7-gliedrigen, vorzugsweise gesättigten, cycloaliphatischen Kohlenwasserstoffrest, an den ein Benzolring ankondensiert sein kann; oder für den Rest

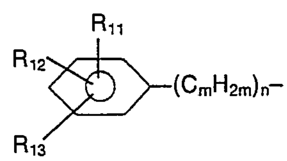 (VI)

steht, wobei

k die Zahlen 0, 1 oder 2,
m die Zahlen 1, 2, 3 oder 4,
n die Zahlen 0 oder 1,
$R_1$ Wasserstoff, $C_1$–$C_4$-Alkyl, Cyano und, wenn k gleich 0 ist, auch Chlor, Brom oder Jod,

$R_2$ und $R_{11}$ Wasserstoff, Halogen, Nitro, gegebenenfalls ein- oder mehrfach durch Fluor oder Chlor substituiertes $C_1$–$C_4$-Alkyl oder -Alkoxy, $C_3$–$C_4$-Alkenyloxy, $C_3$–$C_4$-Alkinyloxy, Amino, $-NH(C_1–C_4\text{-Alkyl})$, $-N(C_1–C_4\text{-Alkyl})_2$, Cyano, Phenyl, $C_3$–$C_6$-Cycloalkyl, Hydroxy($C_1$–$C_4$-Alkyl), $-NH-COR_6$, $-CO_2R_6$, $-CONR_7R_8$, durch Sauerstoff unterbrochenes $C_2$–$C_8$-Alkyl oder

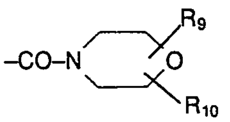 (VII),

$R_3$, $R_4$, $R_{12}$ und $R_{13}$ Wasserstoff, Halogen, $C_1$–$C_4$-Alkyl, $C_1$–$C_4$-Alkoxy, ($C_1$–$C_4$-Alkyl)-S(O)$_p$ (p = 0, 1 oder 2), Hydroxy oder ($C_1$–$C_4$-Acyl)oxy, $R_3/R_4$ und $R_{12}/R_{13}$ jeweils gemeinsam auch Methylendioxy oder Ethylendioxy, gebunden an zwei benachbarte Atome des Phenylrings,

$R_5$ und $R_6$ Wasserstoff oder $C_1$–$C_4$-Alkyl, $R_5$ ausserdem zusammen mit dem C-Atom, an das es gebunden ist, und Y einen 5- bis 7-gliedrigen, vorzugsweise gesättigten, cycloaliphatischen Rest, an den ein Benzolring ankondensiert sein kann,

$R_7$ und $R_8$ $C_1$–$C_4$-Alkyl, $C_3$–$C_7$-Cycloalkyl, Phenyl, Benzyl, Furfuryl, Tetrahydrofurfuryl oder $C_3$–$C_4$-Alkenyl, gemeinsam ausserdem auch eine $C_3$–$C_5$-Alkylenkette, die durch O, $NR_6$, S(O)$_q$ (mit q = 0, 1 oder 2) unterbrochen sein kann, $R_7$ ausserdem Wasserstoff,

$R_9$ und $R_{10}$, die nur vorliegen, wenn $R_7$ und $R_8$ gemeinsam eine Kette darstellen, Wasserstoff oder $C_1$–$C_4$-Alkyl bedeuten,

sowie gegebenenfalls die Säureadditionssalze der vorstehend definierten Verbindungen, mit der Einschränkung, dass A und B nicht beide für einen Rest aus der Gruppe Phenyl, Monohalogenphenyl, Mononitrophenyl und Monoaminophenyl stehen.

2. Verbindungen nach Anspruch 1, in denen X für Sauerstoff steht.

3. Verbindungen nach Anspruch 1 oder 2, worin Q einen Rest der Formel

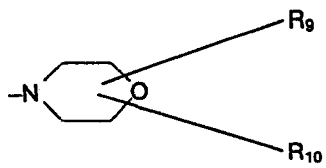 (V)

darstellt, worin $R_9$ und $R_{10}$ die oben angegebene Bedeutung haben.

4. Verbindungen nach Anspruch 1, 2 oder 3, worin A einen in 3- und 4-Stellung in der angegebenen Weise disubstituierten Phenylrest bedeutet.

5. Verbindung nach Anspruch 1, 2, 3 oder 4, in denen $R_1$ Wasserstoff ist.

6. Verbindungen nach Anspruch 3 bis 5, dadurch gekennzeichnet, dass $R_9$ und $R_{10}$ Wasserstoff sind.

7. Fungizide Mittel, gekennzeichnet durch einen Gehalt an einer Verbindung nach Anspruch 1 bis 6, neben üblichen Hilfs- und/oder Trägerstoffen.

8. Verwendung von Verbindungen nach Anspruch 1 bis 6 zur Bekämpfung phytopathogener Pilze, insbesondere falscher Mehltaupilze.

9. Verfahren zur Herstellung von Verbindungen der Formel

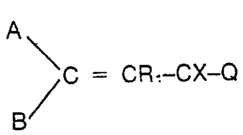 (I),

in der

A für die Gruppe

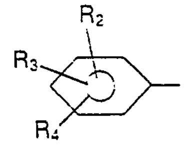 (II),

B für die Gruppe

$$Y-(CR_5=CR_6)_k- \quad (III),$$

Q für eine der Gruppen

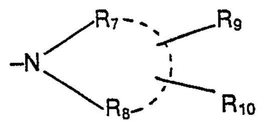 (IV)

und

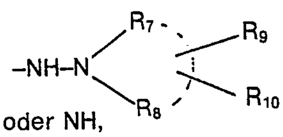 (V)

X für O, S oder NH,

Y für $C_3$–$C_{10}$-Alkyl sowie zusätzlich für $C_1$–$C_2$-Alkyl, wenn k gleich 1 oder 2 ist; oder für substituiertes $C_1$–$C_{10}$-Alkyl oder für einen gegebenenfalls substituierten Rest aus der Gruppe $C_3$–$C_7$-Alkenyl, $C_3$–$C_7$-Cycloalkyl, $C_5$–$C_7$-Cycloalkenyl, Pyridyl, Furyl, Thienyl, α- und β-Naphthyl, wobei die Substituenten Halogen, Nitro, Amino, gegebenenfalls ein- oder mehrfach halogensubstituierte $C_1$–$C_4$-Alkyl- und Alkoxygruppen, NH($C_1$–$C_4$-Alkyl), N($C_1$–$C_4$-Alkyl)$_2$, Phenoxy, Phenylthio oder $C_1$–$C_4$-Alkylthio sind; oder zusammen mit der Gruppe $CR_5$ für einen 5- bis 7-gliedrigen, vorzugsweise gesättigten, cycloaliphatischen Kohlenwasserstoffrest, an den ein Benzolring ankondensiert sein kann; oder für den Rest

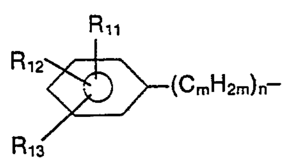 (VI)

steht, wobei

k die Zahlen 0, 1 oder 2,

m die Zahlen 1, 2, 3 oder 4,

n die Zahlen 0 oder 1,

$R_1$ Wasserstoff, $C_1$–$C_4$-Alkyl, Cyano und, wenn k gleich 0 ist, auch Chlor, Brom oder Jod,

$R_2$ und $R_{11}$ Wasserstoff, Halogen, Nitro, gegebenenfalls ein- oder mehrfach durch Fluor oder Chlor substituiertes $C_1$–$C_4$-Alkyl, oder –Alkoxy, $C_3$–$C_4$-Alkenyloxy, $C_3$–$C_4$-Alkinyloxy, Amino, –NH($C_1$–$C_4$-Alkyl), –N($C_1$–$C_4$-Alkyl)$_2$, Cyano, Phenyl, $C_3$–$C_6$-Cycloalkyl, Hydroxy($C_1$–$C_4$-Alkyl), –NH–$COR_6$, –$CO_2R_6$, –$CONR_7R_8$, durch Sauerstoff unterbrochenes $C_2$–$C_8$-Alkyl oder

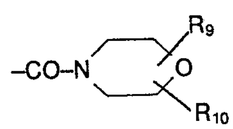 (VII),

$R_3$, $R_4$, $R_{12}$ und $R_{13}$ Wasserstoff, Halogen, $C_1$–$C_4$-Alkyl, $C_1$–$C_4$-Alkoxy, ($C_1$–$C_4$-Alkyl)-S(O)$_p$ ($p = 0$, 1 oder 2), Hydroxy oder ($C_1$–$C_4$-Acyl)oxy, $R_3$/$R_4$ und $R_{12}$/$R_{13}$ jeweils gemeinsam auch Methylendioxy oder Ethylendioxy, gebunden an zwei benachbarte Atome des Phenylrings,

$R_5$ und $R_6$ Wasserstoff oder $C_1$–$C_4$-Alkyl, $R_5$ ausserdem, zusammen mit dem C-Atom, an das es gebunden ist, und Y einen 5- bis 7-gliedrigen, vorzugsweise gesättigten, cycloaliphatischen Rest, an den ein Benzolring ankondensiert sein kann.

$R_7$ und $R_8$ $C_1$–$C_4$-Alkyl, $C_3$–$C_7$-Cycloalkyl, Phenyl, Benzyl, Furfuryl, Tetrahydrofurfuryl oder $C_3$–$C_4$-Alkenyl, gemeinsam ausserdem auch eine $C_3$–$C_5$-Alkylenkette, die durch O, $NR_6$, S(O)$_q$ (mit $q = 0$, 1 oder 2) unterbrochen sein kann, $R_7$ ausserdem Wasserstoff,

$R_9$ und $R_{10}$, die nur vorliegen, wenn $R_7$ und $R_8$ gemeinsam eine Kette darstellen, Wasserstoff oder $C_1$–$C_4$-Alkyl bedeuten,
sowie gegebenenfalls die Säureadditionssalze der vorstehend definierten Verbindungen, mit der Einschränkung, dass A und B nicht beide für einen Rest aus der Gruppe Phenyl, Monohalogenphenyl, Mononitrophenyl und Monoaminophenyl stehen, dadurch gekennzeichnet, dass man

a) eine Acrylsäure der Formel

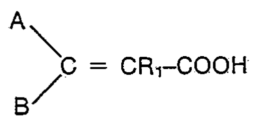 (VIII),

in der A, B und $R_1$ die obige Bedeutung haben, oder eines ihrer gegebenenfalls in situ hergestellten reaktionsfähigen Derivate mit einer Verbindung der Formel

$$H\text{–}Q \qquad (IX),$$

worin Q die obige Bedeutung hat, oder mit einem gegebenenfalls in situ hergestellten N-aktivierten Derivat von IX umsetzt
oder dass man

b) ein Keton der Formel

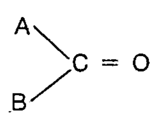 (X),

worin A und B die obige Bedeutung haben, mit einem Phosphonsäurederivat der Formel

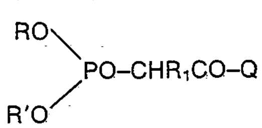 (XI),

worin $R_1$ und Q die obige Bedeutung haben und R und R′, die gleich oder verschieden sein können, für geradkettige oder verzweigte $C_1$–$C_{12}$-Alkylreste, für $C_7$–$C_{12}$-Aralkylreste oder $C_6$–$C_{10}$-Arylreste stehen; wobei die Alkylketten durch 0 oder 5 unterbrochen, die Aryl- und Aralkylreste ein- oder mehrfach durch Halogen, $C_1$–$C_2$-Alkyl oder -Alkoxy im Kern substituiert sein können
oder dass man

c) die Verbindungen der Formel I, in denen X Sauerstoff bedeutet, in einem inerten Lösungsmittel mit Phosphorpentasulfid umsetzt
oder dass man

d) eine Verbindung der Formel

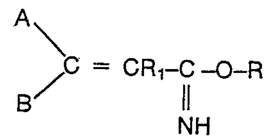 (XII),

worin A, B, R und $R_1$ die obige Bedeutung haben, mit Aminen der Formel IX umsetzt
oder dass man

e) bei einer Verbindung der Formel I, in der k 0 bedeutet, Chlor, Brom oder Jod an die Doppelbindung addiert und dann den entsprechenden Halogenwasserstoff abspaltet
oder dass man

f) eine Verbindung der Formel I, in der $R_1$ Halogen bedeutet, mit Kupfer(I)cyanid umsetzt
oder dass man

g) ein Keton der Formel X mit einem Cyanessigsäurederivat der Formel

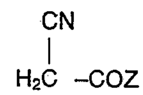 (XIII) ,

in der Z für Q oder OR steht, wobei Q und R wie oben definiert sind, umsetzt
oder dass man

h) Verbindungen der Formel I, in denen A und/oder B niedere bis mittlere Alkoxygruppen oder Benzoylgruppen enthalten, einer Etherspaltung unterwirft und gegebenenfalls dann die freien phenolischen OH-Gruppen acyliert
oder dass man

i) Verbindungen der Formel I, in denen A und/oder B Nitrogruppen enthalten, zu den entsprechenden Aminoverbindungen reduziert und dass man gegebenenfalls vorliegende cis/trans-Isomerengemische gewünschtenfalls auftrennt und/oder dass man nach a) bis i) erhaltene Verbindungen der Formel I mit basischen Gruppen in Säureadditionssalze überführt.

**Claims**

1. Compounds of the general formula

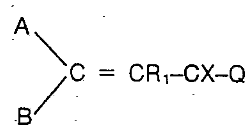 (I),

wherein
A represents the group

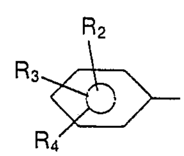 (II),

B represents the group

$$Y\text{–}(CR_5{=}CR_6)k\text{–} \qquad (III),$$

Q represents one of the groups

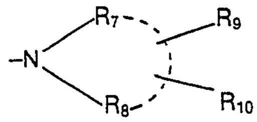 (IV)

and

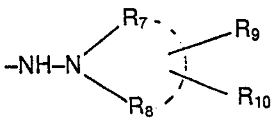 (V)

X represents O, S or NH,

Y represents $C_{3-10}$ alkyl, and also $C_{1-2}$ alkyl if k equals 1 or 2, or represents substituted $C_{1-10}$ alkyl or an optionally substituted moiety from the group comprising $C_{3-7}$ alkenyl, $C_{3-7}$ cycloalkyl, $C_{3-7}$ cycloalkenyl, pyridyl, furyl, thienyl, α- and β-naphthyl, whereby the halogen, nitro and amino substituents are optionally mono- or polyhalogenated $C_{1-4}$ alkyl and alkoxy groups, $-NH(C_{1-4}$ alkyl), $-N(C_{1-4}$ alkyl$)_2$, phenoxy, phenylthio or $C_{1-4}$ alkylthio; or together with the $CR_5$ group represents a preferably saturated 5–7 branch cycloaliphatic hydrocarbon moiety, to which a benzene ring may be condensed; or represents the moiety

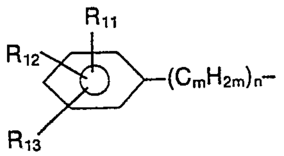 (VI)

wherein

k represents the numbers 0, 1 or 2,

m the numbers 1, 2, 3 or 4,

n the numbers 0 or 1,

$R_1$ hydrogen, $C_{1-4}$ alkyl, cyano and, if k equals 0, also chlorine, bromine or iodine,

$R_2$ and $R_{11}$ represent hydrogen, halogen, nitro, optionally mono- or polyfluorinated or -chlorinated $C_{1-4}$ alkyl or -alkoxy, $C_{3-4}$ alkenyloxy, $C_{3-4}$ alkynyloxy, amino, $-NH(C_{1-4}$ alkyl), $-N(C_{1-4}$ alkyl$)_2$, cyano, phenyl, $C_{3-6}$ cycloalkyl, hydroxy($C_{1-4}$ alkyl), $-NH-COR_6$, $-CO_2R_6$, $-CONR_7R_8$, oxygen-interrupted $C_{1-8}$ alkyl or

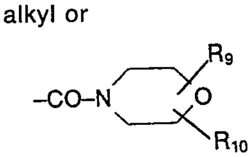 (VII),

$R_3$, $R_4$, $R_{12}$ and $R_{13}$ represent hydrogen, halogen, $C_{1-4}$ alkyl, $C_{1-4}$ alkoxy, ($C_{1-4}$ alkyl)-S(O)$_p$ ( p = 0, 1 or 2), hydroxy or ($C_{1-4}$ acyl)oxy, each $R_3/R_4$ and $R_{12}/R_{13}$ pair also representing methylendioxy or ethylendioxy, linked to two neighbouring atoms of the phenyl ring,

$R_5$ and $R_6$ represent hydrogen or $C_{1-4}$ alkyl, additionally $R_5$ together with the carbon atom to which it is linked and Y also representing a preferably saturated 5–7 branch cycloaliphatic hydrocarbon moiety to which a benzene ring may be condensed,

$R_7$ and $R_8$ each independently represent $C_{1-4}$ alkyl, $C_{3-7}$ cycloalkyl, phenyl, benzyl, furfuryl, tetrahydrofurfuryl or $C_{3-5}$ alkenyl, together also a $C_{3-5}$ alkylene chain that may be interrupted by O, $NR_6$, $S(O)_q$ (with q = 0, 1 or 2), $R_7$ can also represent hydrogen,

$R_9$ and $R_{10}$, which only occur when $R_7$ and $R_8$ together represent a chain, represent hydrogen or $C_{1-4}$ alkyl,

or the acid addition salts of the compounds defined above, provided that A and B do not both represent a moiety from the group comprising phenyl, monohalophenyl, mononitrophenyl and monaminophenyl.

2. Compounds as claimed in claim 1, in which X represents oxygen.

3. Compounds as claimed in claim 1 or 2, in which 0 represents a moiety of the formula

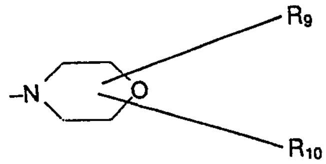 (V)

wherein $R_9$ and $R_{10}$ have the meanings given above.

4. Compounds as claimed in claim 1, 2 or 3, in which A represents a phenyl moiety substituted in the manner indicated in the 3rd or 4th position.

5. Compounds as claimed in claim 1, 2, 3 or 4, in which $R_1$ represents hydrogen.

6. Compounds as claimed in claims 3–5, characterized in that $R_9$ and $R_{10}$ represent hydrogen.

7. Fungicides, characterized in that they contain a compound as claimed in claims 1–6, in addition to the normal adjuvants and/or carriers.

8. The use of compounds as claimed in claims 1–6 for combating phytopathogenic fungi, particularly false mildew fungi.

9. Process for the production of compounds of the formula

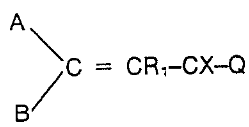 (I).

wherein

A represents the group

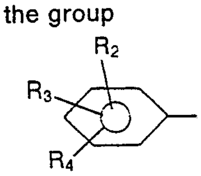 (II),

B represents the group

$$Y-(CR_5=CR_6)_k- \quad \text{(III)},$$

Q represents one of the groups

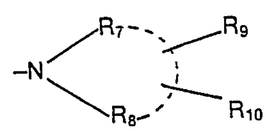 (IV)

and

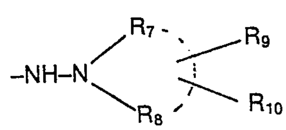 (V)

X represents O, S or NH,

Y represents $C_{3-10}$ alkyl, and also $C_{1-2}$ alkyl if k equals 1 or 2, or represents substituted $C_{1-10}$ alkyl or an optionally substituted moiety from the group comprising $C_{3-7}$ alkenyl, $C_{3-7}$ cycloalkyl, $C_{5-7}$ cycloalkenyl, pyridyl, furyl, thienyl, α- and β-naphthyl, whereby the halogen, nitro and amino substituents are optionally mono- or polyhalogenated $C_{1-4}$ alkyl and alkoxy groups, $-NH(C_{1-4}$ alkyl), $-N(C_{1-4}$ alkyl$)_2$, phenoxy, phenylthio or $C_{1-4}$ alkylthio; or together with the $CR_5$ group represents a preferably saturated 5–7 branch cycloaliphatic hydrocarbon moiety, to which a benzene ring may be condensed; or represents the moiety

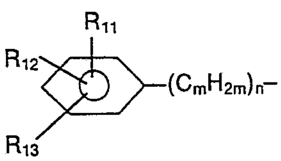 (VI)

wherein

k represents the numbers 0, 1 or 2,

m the numbers 1, 2, 3 or 4,

n the numbers 0 or 1,

$R_1$ hydrogen, $C_{1-4}$ alky, cyano and, if k equals 0, also chlorine, bromine or iodine,

$R_2$ and $R_{11}$ represent hydrogen, halogen, nitro, optionally mono- or polyfluorinated or -chlorinated $C_{1-4}$ alkyl or -alkoxy, $C_{3-4}$ alkenyloxy, $C_{3-4}$ alkynyloxy, amino, $-NH(C_{1-4}$ alkyl), $-N(C_{1-4}$ alkyl$)_2$, cyano, phenyl, $C_{3-6}$ cycloalkyl, hydroxy($C_{1-4}$ alkyl), $-NH-COR_6$, $-CO_2R_6$, $-CONR_7T_8$, oxygen-interrupted $C_{2-8}$ alkyl or

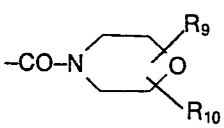 (VII),

$R_3$, $R_4$, $R_{12}$ and $R_{13}$ represeent hydrogen, halogen, $C_{1-4}$ alkyl, $C_{1-4}$ alkoxy, ($C_{1-4}$ alkyl)-$S(O)_p$ (p = O 1 or 2), hydroxy or ($C_{1-4}$ acyl)oxy, each $R_3/R_4$ and $R_{12}/R_{13}$ pair also representing methylenedioxy or ethylenedioxy, linked to two neighbouring atoms of the phenyl ring,

$R_5$ and $R_6$ represent hydrogen or $C_{1-4}$ alkyl, additionally $R_5$ together with the carbon atom to which it is linked and Y also representing a preferably saturated 5–7 branch cycloaliphatic hydrocarbon moiety to which a benzene ring may be condensed.

$R_7$ and $R_8$ each independently represent $C_{1-4}$ alkyl, $C_{3-7}$ cycloalkyl, phenyl, benzyl, furfuryl, tetrahydrofurfuryl or $C_{3-5}$ alkenyl, together also a $C_{3-5}$ alkylene chain that may be interrupted by O, $NR_6$, $S(O)_q$ (with q = 0, 1 or 2), $R_7$ can also represent hydrogen,

$R_9$ and $R_{10}$, which only occur when $R_7$ and $R_8$ together represent a chain, represent hydrogen or $C_{1-4}$ alkyl,

or the acid addition salts of the compounds defined above, provided that A and B do not both represent a moiety from the group comprising phenyl, monohalophenyl, mononitrophenyl and monoaminophenyl, characterized in that

a) an acrylic acid of the formula

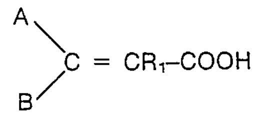 (VIII),

wherein A, B and $R_1$ have the meanings given above, or one of their reactive derivatives, optionally produced in situ, is reacted with a compound of the formula

$$H-Q \qquad (IX),$$

wherein Q has the meaning given above, or with a N-activated derivative of IX, optionally produced in situ,

or

b) a ketone of the formula

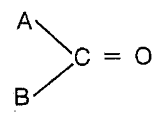 (X)

wherein A and B have the meanings given above, is reacted with a phosphonic acid derivative of the formula

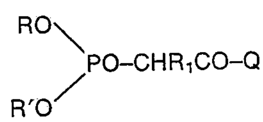 (XI)

wherein $R_1$ and Q have the meanings given above and R and R′, which may be the same or different, represent unbranched or branched $C_{1-12}$ alkyl moieties, $C_{7-12}$ aralkyl moieties or $C_{6-10}$ aryl moieties; whereby the alkyl chains may be interrupted by O or S, and the aryl moieties and aralkyl moieties may be mono- or polysubstituted by halogen, $C_{1-2}$ alkyl or -alkoxy in the nucleus.

or

c) compounds of formula I, wherein X represents oxygen, are reacted with phosphorus pentasulphide in an inert solvent,

or

d) a compound of the formula

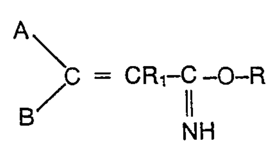 (XII),

wherein A, B, R and $R_1$ have the meanings given above, is reacted with amines of formula IX,

or

e) in a compound of formula I, wherein k represents 0, chlorine, bromine or iodine is added to the double bond and the corresponding hydrohalogen is split off

or

f) a compound of formula I, in which $R_1$ represents halogen, is reacted with copper(I) cyanide or

g) a ketone of formula X is reacted with a cyanoacetic acid of the formula

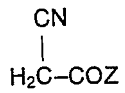 (XIII),

in which Z represents Q or OR, whereby Q and R are defined as above,
or

h) compounds of formula I, wherein A and/or B comprises lower-to-middle alkoxy groups or benzoyl groups, are subjected to ether cleavage and the free phenolic OH groups are then optionally acylated
or

i) compounds of formula I, wherein A and/or B comprise nitro groups, are reduced to the corresponding amino compounds
and that any cis/trans isomer mixtures are, if desired, split and/or that compounds of formula I obtained according to a)–i) are converted into acid addition salts by means of basic groups.

**Revendications**

1. Composés répondant à la formule

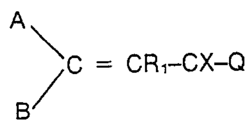 (I),

dans laquelle
A désigne le groupe

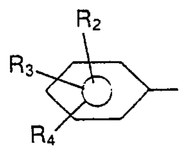 (II),

B le groupe

$$Y-(CR_5=CR_6)_k- \quad (III)$$

Q un des groupes

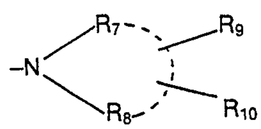 (IV)

et

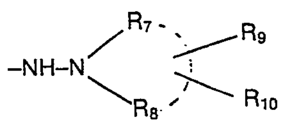 (V)

X désigne O, S ou NH,

Y désigne un groupe alkyle en $C_3$ à $C_{10}$, et en outre un groupe alkyle en $C_1$ à $C_2$ lorsque k est égal à 1 ou 2 ou un groupe alkyle en $C_1$ à $C_{10}$ substitué ou un radical substitué le cas échéant, choisi parmi les groupes alcényle en $C_3$ à $C_7$, cycloalkyle en $C_3$ à $C_7$, cycloalcényle en $C_5$ à $C_7$, pyridyle, furyle, thiényle, α- ou β-naphtyle, les substituants étant un atome d'halogène, un groupe nitro, amino, un groupe alkyle et alcoxy en $C_1$ à $C_4$ substitué le cas échéant une ou plusieurs fois par des halogènes, –NH(alkyl en $C_1$ à $C_4$), –N(alkyl en $C_1$ à $C_4$)$_2$, phénoxy, phénylthio ou alkylthio en $C_1$ à $C_4$; ou peut être condensé à un cycle benzénique avec le groupe $CR_5$ pour donner un radical hydrocarboné cycloaliphatique en $C_5$ à $C_7$, de préférence saturé; ou le radical

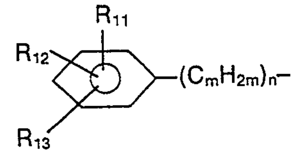 (VI)

dans lequel

k désigne les nombres 0, 1 ou 2,

m désigne les nombres 1, 2, 3 ou 4,

n désigne les nombres 0 ou 1,

$R_1$ désigne l'hydrogène, un groupe alkyle en $C_1$ à $C_4$, cyano, et lorsque k est égal à 0, également le chlore, le brome ou l'iode,

$R_2$ et $R_{11}$ désignent un atome d'hydrogène, un atome d'halogène, un groupe nitro, un groupe alkyle ou alcoxy en $C_1$ à $C_4$ substitué le cas échéant une ou plusieurs fois par le fluor ou le chlore, un groupe alcényloxy en $C_3$ à $C_4$, alcynyloxy en $C_3$ à $C_4$, amino, –NH(alkyl en $C_1$ à $C_4$), –N(alkyl en $C_1$ à $C_4$)$_2$, cyano, phényle, cycloalkyle en $C_3$ à $C_6$, hydroxy(alkyl en $C_1$ à $C_4$), $-NH-COR_6$, $-CO_2R_6$, $-CONR_7R_8$, un groupe alkyle en $C_2$ à $C_8$ interrompu par un atome d'oxygène ou

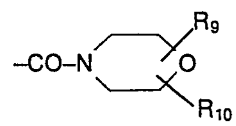 (VII),

$R_3$, $R_4$, $R_{12}$ et $R_{13}$ désignent un atome d'hydrogène, un atome d'halogène, un groupe alkyle en $C_1$ à $C_4$, alcoxy en $C_1$ à $C_4$, (alkyl en $C_1$ à $C_4$)-S(O)p (p = 0, 1 ou 2), hydroxy ou (acyl en $C_1$ à $C_4$)oxy, $R_3/R_4$ et $R_{12}/R_{13}$ désignent également à chaque fois en commun un groupe méthylènedioxy ou éthylènedioxy, lié à deux atomes voisins du cycle phényle.

$R_5$ et $R_6$ désignent un atome d'hydrogène ou un groupe alkyle en $C_1$ à $C_4$, $R_5$ désigne en outre, avec l'atome de C auquel il est lié et Y, un radical cycloaliphatique à 5 à 7 maillons, de préférence saturé, sur lequel un cycle benzénique peut être condensé.

$R_7$ et $R_8$ désignent un groupe alkyle en $C_1$ à $C_4$, cycloalkyle en $C_3$ à $C_7$, phényle, benzyle, furfuryle, tétrahydrofurfuryle ou alcényle en $C_3$ à $C_4$, et en outre conjointement une chaîne alkylène en $C_3$ à $C_5$, qui peut être interrompue par O, $NR_6$, $S(O)_q$ (q = 0, 1 ou 2), $R_7$ désigne en outre un atome d'hydrogène,

$R_9$ et $R_{10}$, qui ne sont présents que lorsque $R_7$ et $R_8$ représentent en commun une chaîne, désignent un atome d'hydrogène ou un groupe alkyle en $C_1$ à $C_4$,
ainsi que le cas échéant les sels d'addition aux acides des composés précédemment définis, avec cette limitation que A et B ne représentent pas tous deux un radical du groupe des radicaux phényle, monohalogénophényle, mononitrophényle et monoaminophényle.

2. Composés selon la revendication 1, dans lesquels X désigne l'oxygène.

3. Composés selon les revendications 1 ou 2, dans lesquels Q désigne un radical répondant à la formule

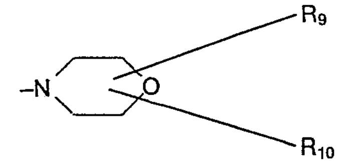

(V)

dans laquelle $R_9$ et $R_{10}$ ont la signification indiquée ci-dessus.

4. Composés selon les revendications 1, 2 ou 3, dans lesquels A désigne un radical phényle disubstitué en position 3 et 4 de la manière indiquée.

5. Composés selon les revendications 1, 2, 3 ou 4, dans lesquels $R_1$ est un atome d'hydrogène.

6. Composés selon les revendications 3 à 5, caractérisés par le fait que $R_9$ et $R_{10}$ sont des atomes d'hydrogène.

7. Agents fongicides, caractérisés par le fait qu'ils contiennent un composé selon les revendications 1 à 6, en plus de substances auxiliaires et/ou de supports usuels.

8. Utilisation de composés selon les revendications 1 à 6 pour la lutte contre les champignons phytopathogènes, en particulier contre les champignons du mildiou.

9. Procédé de préparation de composés répondant à la formule

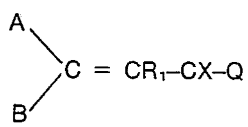

(I),

dans laquelle

A désigne le groupe

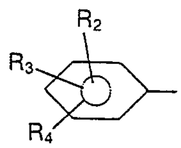

(II),

B désigne le groupe

$$Y-(CR_5=CR_6)_k- \quad (III),$$

Q désigne un des groupes

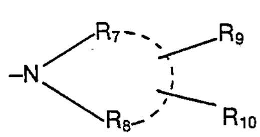

(IV)

et

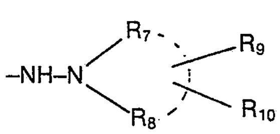

(V)

X désigne O, S ou NH,

Y désigne un groupe alkyle en $C_3$ à $C_{10}$, et en outre un groupe alkyle en $C_1$ à $C_2$ lorsque k est égal à 1 ou 2; ou un groupe alkyle en $C_1$ à $C_{10}$ substitué ou un radical substitué le cas échéant, choisi parmi les groupes alcényle en $C_3$ à $C_7$, cycloalkyle en $C_3$ à $C_7$, cycloalcényle en $C_5$ à $C_7$, pyridyle, furyle, thiényle, α- ou β-naphtyle les substituants étant un atome d'halogène, un groupe nitro, amino, un groupe alkyle et alcoxy en $C_1$ à $C_4$ substitué le cas échéant une ou plusieurs fois par des halogènes, –NH(alkyl en $C_1$ à $C_4$), –N(alkyl en $C_1$ à $C_4)_2$, phénoxy ou alkylthio en $C_1$ à $C_4$; ou désigne avec le groupe $CR_5$ un radical hydrocarboné cycloaliphatique à 5 à 7 maillons, de préférence saturé, sur lequel un cycle benzénique peut être condensé; ou le radical

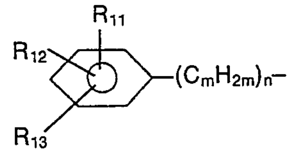

(VI)

dans lequel

k désigne les nombres 0, 1 ou 2,

m désigne les nombres 1, 2, 3 ou 4,

n désigne les nombres 0 ou 1,

$R_1$ désigne l'hydrogène, un groupe alkyle en $C_1$ à $C_4$, cyano, et lorsque k est égal à 0, également le chlore, le brome ou l'iode,

$R_2$ et $R_{11}$ désignent un atome d'hydrogène, un atome d'halogène, un groupe nitro, un groupe alkyle ou alcoxy en $C_1$ à $C_4$ substitué le cas échéant une ou plusieurs fois par le fluor ou le chlore, un groupe alcényloxy en $C_3$ à $C_4$, alcynyloxy en $C_3$ à $C_4$, amino, –NH(alkyl en $C_1$ à $C_4$), –N(alkyl en $C_1$ à $C_4)_2$, cyano, phényle, cycloalkyle en $C_3$ à $C_6$,hydroxy(alkyl en $C_1$ à $C_4$), $-NH-COR_6$, $-CO_2R_6$, $-CONR_7R_8$, un groupe alkyle en $C_2$ à $C_8$ interrompu par un atome d'oxygène ou

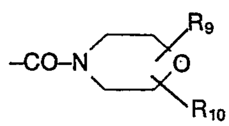

(VII),

$R_3$, $R_4$, $R_{12}$ et $R_{13}$ désignent un atome d'hydrogène, un atome d'halogène, un groupe alkyle en $C_1$ à $C_4$, alcoxy en $C_1$ à $C_4$, (alkyl en $C_1$ à $C_4$)-$S(O)_p$ (p = 0, 1 ou 2), hydroxy ou (acyl en $C_1$ à $C_4$)oxy, $R_3/R_4$ et $R_{12}/R_{13}$ désignent également à chaque fois en commun un groupe méthylènedioxy ou éthylènedioxy, lié à deux atomes voisins du cycle phényle.

$R_5$ et $R_6$ désignent un atome d'hydrogène ou un groupe alkyle en $C_1$ à $C_4$, $R_5$ désigne en outre, avec l'atome de C auquel il est lié et Y, un radical cycloaliphatique à 5 à 7 maillons, de préférence saturé, sur lequel un cycle benzénique peut être condensé.

$R_7$ et $R_8$ désignent un groupe alkyle en $C_1$ à $C_4$, cyclo-alkyle en $C_3$ à $C_7$, phényle, benzyle, furfuryle, tétrahydrofurfuryle ou alcényle en $C_3$ à $C_4$, et en outre conjointement une chaîne alkylène en $C_3$ à $C_5$, qui peut être interrompue par O, $NR_6$, $S(O)_q$ (q = 0, 1 ou 2), $R_7$ désigne en outre un atome d'hydrogène,

$R_9$ et $R_{10}$, qui ne sont présents que lorsque $R_7$ et $R_8$ représentent en commun une chaîne, désignent un atome d'hydrogène ou un groupe alkyle en $C_1$ à $C_4$,
ainsi que le cas échéant les sels d'addition aux acides des composés précédemment définis, avec cette limitation que A et B ne représentent pas tous deux un radical du groupe des radicaux phényle, monohalogénophényle, mononitrophényle et monoaminophényle, caractérisé par le fait que

a) On fait réagir un acide acrylique répondant à la formule

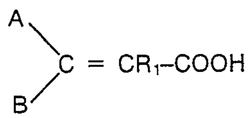
(VIII)

dans laquelle A, B et $R_1$ ont la signification ci-dessus, ou un de leurs dérivés réactifs préparés le cas échéant in situ, avec un composé répondant à la formule

$$H{-}Q \qquad (IX)$$

dans laquelle Q a la signification ci-dessus, ou avec un dérivé N-activé de IX préparé le cas échéant in situ
ou par le fait qu'on fait réagir

b) une cétone répondant à la formule

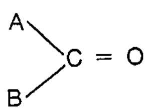
(X)

dans laquelle A et B ont la signification ci-dessus, avec un dérivé de l'acide phosphonique répondant à la formule

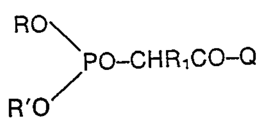
(XI)

dans laquelle $R_1$ et Q ont la signification ci-dessus et R et R′, qui peuvent être identiques ou différents, désignent des radicaux alkyle en $C_1$ à $C_{12}$ linéaires ou ramifiés, des radicaux aralkyle en $C_7$ à $C_{12}$ ou des radicaux aryle en $C_6$ à $C_{10}$; les chaînes alkyle pouvant être iterrompues par O ou S, les radicaux aryle et aralkyle pouvant être substitués une ou plusieurs fois dans le noyau par un atome d'halogène, un radical alkyle ou alcoxy en $C_1$ à $C_2$ ou par le fait qu'on fait réagir

c) les composés répondant à la formule I, dans lesquels X désigne un atome d'oxygène, dans un solvante inerte avec du pentasulfure de phosphore ou par le fait qu'on fait réagir

d) un composé répondant à la formule

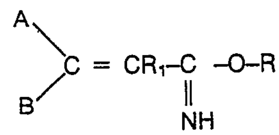
(XII),

dans laquelle A, B, R et $R_1$ ont la signification ci-dessus, avec des amines répondant à la formule IX
ou par le fait que

e) dans un composé répondant à la formule I, dans laquelle k désigne un atome d'oxygène, on ajoute du chlore, du brome ou de l'iode sur la double liaison, puis on élimine l'acide halohydrique correspondant
ou par le fait qu'on fait réagir

f) un composé répondant à la formule I, dans laquelle $R_1$ désigne un atome d'halogène, avec du cyanure de cuivre (I)
ou par le fait qu'on fait réagir

g) une cétone répondant à la formule X avec un dérivé de l'acide cyanacétique répondant à la formule

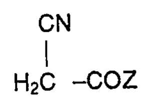
(XIII)

dans laquelle Z désigne Q ou OR, Q et R étant tels que définis ci-dessus
ou par le fait que

h) on soumet des composés répondant à la formule I, dans lesquels A et/ou B contiennent des groupes alcoxy inférieurs à moyens ou des groupes benzoyle, à une élimination de l'éther, puis par le fait qu'on acyle les groupes OH phénoliques libres
ou par le fait que

i) on réduit des composés répondant à la formule I, dans lesquels A et/ou B contiennent des groupes nitro, en les composés amino correspondants
et par le fait qu'on sépare si on le désire les mélanges d'isoméres cis-trans présents et/ou par le fait qu'on transforme les composés répondant à la formule I obtenus selon a) à i), contenant des groupes basiques, en les sels d'addition aux acides.